(19) Europäisches Patentamt
European Patent Office
Office européen des brevets

(11) Veröffentlichungsnummer: **0 254 685 B1**

(12) **EUROPÄISCHE PATENTSCHRIFT**

(45) Veröffentlichungstag der Patentschrift: **13.04.94**

(21) Anmeldenummer: **87810406.6**

(22) Anmeldetag: **20.07.87**

(51) Int. Cl.[5]: **C07H 23/00**, C07F 7/28, //C07C229/00,C07C227/00, C07C303/00,C07C307/00, C07C309/00,C07C311/00, C07F7/10,C07F7/18,C07C33/30, C07C33/025,C07C205/00

# (54) Komplexe mit optisch aktiven Zuckerliganden, Verfahren zu deren Herstellung und deren Verwendung.

(30) Priorität: **23.07.86 CH 2941/86**

(43) Veröffentlichungstag der Anmeldung:
**27.01.88 Patentblatt 88/04**

(45) Bekanntmachung des Hinweises auf die Patenterteilung:
**13.04.94 Patentblatt 94/15**

(84) Benannte Vertragsstaaten:
**AT BE CH DE ES FR GB GR IT LI LU NL SE**

(56) Entgegenhaltungen:
**EP-A- 0 173 142**

**TETRAHEDRON LETTERS, Nr. 41, 1969, Seiten 3657-3660, Pergamon Press, GB; T.D. INCH et al.: "Asymmetric synthesis. Part IV. The stereoselectivity of grignard-carbonyl"**

(73) Patentinhaber: **CIBA-GEIGY AG**
**Klybeckstrasse 141**
**CH-4002 Basel(CH)**

(72) Erfinder: **Riediker, Martin, Dr.**
**Gstaltenrainweg 75**
**CH-4125 Riehen(CH)**
Erfinder: **Lang, Robert Werner, Dr.**
**Hagenbachweg 10**
**CH-4133 Pratteln(CH)**
Erfinder: **Duthaler, Rudolf, Dr.**
**Girenhaldenweg 17**
**Ch-4126 Bettingen(CH)**
Erfinder: **Herold, Peter, Dr.**
**Eichenstrasse 53**
**CH-4054 Basel(CH)**
Erfinder: **Oertle, Konrad, Dr.**
**Vogesenstrasse 10**
**CH-4106 Therwil(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**CHEM. BER., Band 118, 1985, Seiten 3673-3682, VHC Verlagsgesellschaft mbH, Weinheim, DE; D. SEEBACH et al.: "Enantioselektive Addition von Arylgruppen an aromatische Aldehyde mit Aryltitan-Binaphthol-Derivaten"**

**CHEMICAL ABSTRACTS, Band 89, Nr. 23, 4. Dezember 1978, Seite 666, Zusammenfassung Nr. 197884h, Columbus, Ohio, US; E. ZARA-KACZAN et al.: "One-step synthesis of 2,3,4-triacetyllevoglucosan from 1,2,3,4-tetraacetyl-6-trityl-beta-D-glucopyranose using titanium tetrachloride"**

Erfinder: **Bold, Guido, Dr.**
**Hauptstrasse 125**
**CH-5262 Frick(CH)**

## Beschreibung

Die Erfindung betrifft Titan-, Zirkon- oder Hafniumkomplexe mit optisch aktiven Zucherliganden, einem auf Carbonylgruppen oder carbonylanaloge Gruppen übertragbaren Rest und gegebenenfalls einem achiralen Rest, ein Verfahren zu deren Herstellung und deren Verwendung als enantioselektive Reaktanden für Aldehyde, Ketone und N-substituierte Imine.

T.D. Inch et al. beschreiben in Tetrahedron Letters, No. 41, S. 3657-3660 (1969), dass die Umsetzung von prochiralen Ketonen mit chiralen Magnesium-Grignard-Zuckerkomplexen chirale sekundäre Alkohole ergibt. Die Stereoselektivität der Reaktion ist relativ gering. So betragen die optischen Ausbeuten meistens um 25 % und nur in zwei Fällen werden optische Ausbeuten von 65 bzw. 70 % erzielt.

Aus Helvetica Chimica Acta, Vol. 64, Fasc. 7, No. 243, S. 2485-2488 (1981), Organometalics 3, S. 1716-1717 (1984) und Chem. Ber. 118, 3673-3682 (1985) ist bekannt, dass Titanverbindungen mit einer übertragbaren Gruppe und einem von einer chiralen Mono- oder Dihydroxyverbindung abgeleiteten Rest mit Aldehyden zu sekundären Alkoholen umgesetzt werden können. Aliphatische chirale Hydroxyverbindungen als Liganden führen zu nur mässigen Stereoselektivitäten, während mit chiralem Binaphthol als Ligand hohe optische Ausbeuten erzielt werden können. Die verwendeten chiralen Liganden sind schwer zugänglich und teuer.

Es besteht ein Bedürfnis an preiswerten chiralen Organotitan-reagenzien mit hohen Stereoselektivitäten bei der Reaktion mit prochiralen Aldehyden, Ketonen und Iminen, deren chirale Liganden sich von in technischen Mengen leicht zugänglichen, z.B. von natürlichen chiralen Hydroxyverbindungen ableiten.

Ein Gegenstand der Erfindung sind Verbindungen der Formeln I und Ia

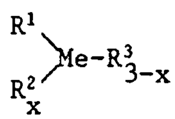 (I) und 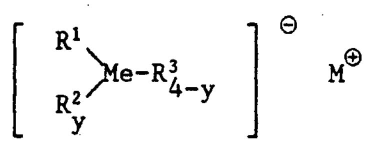 (Ia),

worin

Me für vierwertiges Titan, Zirkonium oder Hafnium steht,

$R^1$ lineares oder verzweigtes Alkyl, Alkenyl, Alkinyl, gegebenenfalls durch $C_1$-$C_4$-Alkyl substituiertes Cycloalkyl oder Cycloalkenyl, Aryl, Alkaryl, Aralkyl, Alkaralkyl, Aralkenyl, Alkaralkenyl, Aralkinyl oder Alkaralkinyl bedeuten, die gegebenenfalls ein- oder mehrfach durch $(C_6H_5)_2P$-, $(R^5O)_2P(O)$-, $R^5_3$ Si-, $R^5_3$ SiO-, $R^5SO_2$-, -S-$C_2$-$C_4$-Alkylen-S-, -O-$C_2$-$C_4$-Alkylen-O-, wobei $R^5$ für Phenyl, Benzyl oder $C_1$-$C_8$-Alkyl steht, Cyano, F, Nitro, $C_1$-$C_{12}$-Alkylthio, $C_1$-$C_{12}$-Alkoxy, Sekundäramino oder -$COR^4$, worin $R^4$ der Rest eines einwertigen Alkohols ist, substituiert sind; oder einen Rest eines Enols, Enamins oder Enhydrazins bedeutet;

$R^2$ gegebenenfalls durch Alkyl, Alkenyl, Alkoxy, Cycloalkyl, Aryl, Aralkyl, Trialkoxysilyl, Trialkylsilyl oder Halogen substituiertes Cyclopentadienyl, Alkoxy, Cycloalkoxy, Cycloalkylalkoxy, Aryloxy, Aralkyloxy, Alkylthio, Arylthio, Aralkylthio; Halogen, Pseudohalogen, Acyloxy, Acylamino oder Trialkylsilyloxy darstellt,

$R^3$ der um ein Hydroxyl-, Thiol- oder Aminwasserstoffatom verminderte Rest eines geschützten, monohydroxyfunktionellen, monothiolfunktionellen oder monoaminfunktionellen, optisch aktiven Zuckers, Thiozuckers oder Aminozuckers, oder deren Derivaten aus der Gruppe der Zuckeralkohole; Ester einer Zuckersäure, Aldozuckersäure oder Ketozuckersäure; Aminozucker, Zuckermercaptale oder Desoxyzucker ist,

x für 0, 1 oder 2 und y für 0, 1, 2 oder 3 und

$M^⊕$ für $Li^⊕$, $Na^⊕$, $K^⊕$, $MgY^⊕$, $ZnY^⊕$, $CdY^⊕$, $HgY^⊕$, $CuY^⊕$ oder quaternäres Ammonium stehen, worin Y Halogen bedeutet.

Alle nachfolgenden Bevorzugungen können zu beliebigen Gruppen aus mindestens zwei bevorzugten Ausführungsformen kombiniert werden.

Bevorzugt sind die Verbindungen der Formel I. Geschützt und mono-hydroxy-, monothiol-oder monoaminofunktionell bedeutet, dass die Hydroxy-, Thiol- oder Aminogruppen des Zuckers bzw. Zuckerderivats bis auf eine OH-, SH-oder NH-Gruppe geschützt sind. Bevorzugt sind monohydroxyfunktionelle Zucker bzw. Zuckerderivate. Me bedeutet bevorzugt Ti.

Optisch aktiv bedeutet einen Gehalt an überwiegend einem Enantiomeren, vorzugsweise ein im wesentlichen reines Enantiomeres.

$R^1$ ist eine auf Carbonyl- und Iminverbindungen übertragbare Gruppe. $R^1$ enthält als Alkyl bevorzugt 1 bis 18 und besonders 1-12 C-Atome. Beispiele sind Methyl, Ethyl, n- oder i-Propyl, n-, i- oder t-Butyl, Pentyl, Hexyl, Heptyl, Octyl, Nonyl, Decyl, Undecyl, Dodecyl, Tetradecyl, Hexadecyl, Octadecyl und

Eicosyl. Besonders bevorzugt sind Methyl, Ethyl, Propyl und Butyl, die wie zuvor angegeben substituiert sein können.

$R^1$ als Alkenyl enthält bevorzugt 2 bis 12, besonders 2 bis 6 C-Atome. Es kann sich sowohl um Alkenylalkyl der Formel

$$(C_nH_{2n-1})-C_mH_{2m}- ,$$

worin n eine Zahl von 2 bis 12, vorzugsweise 2 bis 6 und m eine Zahl von 1 bis 6, vorzugsweise 1 oder 2, als auch um Alkylvinyl mit bevorzugt 1 bis 10 C-Atomen in der Alkylgruppe handeln. Beispiele sind: Allyl, 1-Methylallyl, 2-Methylallyl, But-2-en-4-yl, But-1-en-3-yl, Pent-3-en-5-yl, Pent-1-en-3-yl, Hex-4-en-6-yl, Hex-2-en-4-yl, Hept-2-en-1-yl), Hept-3-en-5-yl, Oct-6-en-8-yl, Oct-2-en-4-yl, Non-2-en-2-yl, Dec-8-en-10-yl, Dodec-3-en-12-yl, Vinyl, Crotonyl, n-But-1-en-1-yl, But-2-en-3-yl, Pent-1-en-2-yl, Hex-1-en-1-yl. Bevorzugt ist $R^1$ Vinyl oder Allyl. Besonders bevorzugt ist $R^1$ Allyl.

$R^1$ als Alkinyl enthält bevorzugt 2-12, besonders 2-6 C-Atome. Die Alkinylgruppe kann sich sowohl in der Kohlenstoffkette befinden oder endständig sein. Beispiele sind: Ethinyl, Prop-2-in-3-yl, Prop-1-in-3-yl, But-1-in-4-yl, But-3-in-4-yl, Pent-4-in-5-yl, Pent-3-in-5-yl, Pent-2-in-4-yl, Pent-1-in-5-yl, Hex-1-in-6-yl. Bevorzugt sind Ethinyl und Propargyl.

$R^1$ als Cycloalkyl und Cycloalkenyl enthält bevorzugt 3-8, besonders 3-6 und insbesondere 5 oder 6 Ring-C-Atome und kann durch $C_1$-$C_4$-Alkyl substituiert sein. Beispiele sind Cyclopropyl, Cyclopropenyl, Cyclobutyl, Cyclobutenyl, Cyclopentyl, Cyclohexyl, Cyclopent-1-en-3-yl, Cyclopent-1-en-1-yl, Cyclohex-1-en-3-yl, Cyclohex-2-en-1-yl, Cyclohept-1-en-3-yl, Cyclooct-1-en-3-yl.

$R^1$ als Aryl enthält bevorzugt 6-12 C-Atome und ist bevorzugt Naphthyl und besonders Phenyl.

$R^1$ als Alkaryl en hält bevorzugt 7 bis 16 C-Atome. Das Aryl ist bevorzugt Phenyl. Das Aryl ist bevorzugt Phenyl. Beispiele sind: Methylphenyl, Dimethylphenyl, Ethylphenyl, n- und i-Propylphenyl, n-, i- und t-Butylphenyl, Di-t-Butylphenyl, Hexylphenyl, Octylphenyl, Decylphenyl.

$R^1$ als Aralkyl enthält bevorzugt 7 bis 16, besonders 7-10 C-Atome. Das Aryl ist bevorzugt Phenyl. Beispiele sind Benzyl, 1- oder 2-Phenylethyl, 1- oder 2-Phenylpropyl, Phenylbutyl, Phenylpentyl und Phenylhexyl. Bevorzugt sind Benzyl und 1- oder 2-Phenylethyl.

Als Alkaralkyl kann $R^1$ bevorzugt 8-16 C-Atome enthalten. Das Aryl ist bevorzugt Phenyl. Beispiele sind Methylbenzyl, Ethylbenzyl, n-oder i-Propylbenzyl, Dimethylbenzyl, n-, i- oder t-Butylbenzyl, Di-t-Butylbenzyl, Hexylbenzyl, Nonylbenzyl, 1-Methylphenyleth-2-yl, 2-Methylphenyleth-2-yl, 1-Methylphenylprop-3-yl.

$R^1$ als Aralkenyl enthält bevorzugt 8 bis 16 C-Atome. Das Aryl ist bevorzugt Phenyl. Beispiele sind Phenylvinyl, 1-Phenyl-prop-1-en-3-yl, 2-Phenyl-prop-2-en-1-yl, 3-Phenyl-prop-1-en-3-yl, Phenylbutenyl, Phenylpentenyl, Phenyhexenyl.

$R^1$ als Alkaralkenyl enthält bevorzugt 9 bis 16 C-Atome. Das Aryl ist bevorzugt Phenyl. Beispiele sind Methylphenylvinyl, Ethylphenylvinyl, Dimethylphenylvinyl, 1-Methylphenyl-prop-2-en-3-yl, 2-Methylphenyl-prop-2-en-3-yl.

$R^1$ als Aralkinyl enthält bevorzugt 8 bis 16 C-Atome. Das Aryl ist bevorzugt Phenyl. Beispiele sind Phenylethinyl, Phenylpropinyl, 1-Phenyl-but-3-in-4-yl.

$R^1$ als Alkaralkinyl enthält bevorzugt 9 bis 16 C-Atome. Das Aryl ist bevorzugt Phenyl. Beispiele sind Methylphenylethinyl und Methylphenylpropargyl.

Einige Beispiele für Reste $R^1$ sind $-CH_2P(O)(OR^5)_2$, Vinyl, Allyl und substituierte Allylreste wie z.B.
$-CH_2CH=CH-P(C_6H_5)$,
$-CH(SR^5)CH=CH-SiR^5_3$ ,

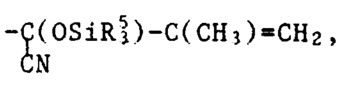

$-CH_2-CH=CH-SO_2-R^5$, $-CH_2-CH=CH-OR'$ und

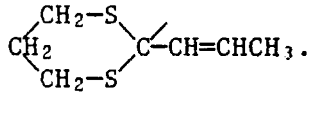

$R^5$ kann die vorher angegebene Bedeutung haben. Bei $R'$ kann es sich beispielsweise um $C_1$-$C_8$-Alkyl, Methoxymethyl, Methoxyisopropyl, 1-Trimethylsilyl-eth-2-yl oder -$SiR^5_3$ handeln.

Der Enolrest $R^1$ ist über das Enolsauerstoffatom an das Metall Me gebunden. Es kann sich um lineare oder cyclische Enole handeln. Der Enolrest $R^1$ enthält bevorzugt bis zu 20, besonders 2 bis 16 C-Atome. Die cyclischen Enole können 3-8, besonders 4-6 Ringatome handeln, wobei der Ring aus Atomen der Gruppe C, O, S und N gebildet sein kann. Der Enolrest $R^1$ kann z.B. der allgemeinen Formel

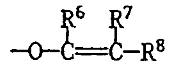

entsprechen. $R^6$ kann z.B. H, gegebenenfalls substituiertes $C_1$-$C_8$-Alkyl, $C_1$-$C_8$-Alkoxy, $C_1$-$C_8$-Alkylthio, Cycloalkyl mit 3-6 Ring-C-Atomen Phenyl, Benzyl, Phenoxy, Phenylthio, Benzyloxy, Benzylthio, Sekundäramino, -$OB(OC_1$-$C_8$-$Alkyl)_2$, -$OSn(R^9)_3$ mit $R^9$ gleich $C_1$-$C_8$-Alkyl, Phenyl oder Benzyl, -$OTi(R^9)_3$, -OLi, -OSi-$(R^5)_3$ sein. In einer bevorzugten Ausführungsform ist $R^1$ 1-(t-butyloxy)vinyl-1-oxyl. $R^7$ und $R^8$ können unabhängig für H, F oder gegebenenfalls substituiertes $C_1$-$C_8$-Alkyl, $C_1$-$C_8$-Alkoxy, $C_1$-$C_8$-Alkylthio, Cycloalkyl mit 3 bis 6 Ring-C-Atomen, Phenyl, Benzyl, Phenoxy, Phenylthio, Benzyloxy, Benzylthio, Sekundäramino oder -$OSi(R^5)_3$ stehen. Als Substituenten kommen die für $R^1$ genannten Substituenten in Frage. Das Sekundäramino kann $C_1$-$C_8$-Alkylgruppen, Phenyl, Benzyl, oder ($R^5_3$ )Si-Gruppen enthalten. Es kann sich auch um heterocyclische Sekundäraminogruppen handeln, die vorzugsweise 4-6 Ringatome aus der Gruppe C, O, S, Si und N enthalten. $R^6$ und $R^7$ bzw. $R^7$ und $R^8$ zusammen können zusammen mit den C-Atomen, an die sie gebunden sind, eine 3-8, vorzugsweise 4-6-gliedrigen carbocyclischen oder heterocyclischen Ring bilden, wobei der heterocyclische Ring Atome aus der Gruppe C, O, S und N enthalten kann. Die Ringe können wie Zuvor für $R^1$ definiert substituiert sein. Eine bevorzugte Gruppe sind Enole von Glycinderivaten, die z.B. der Formel

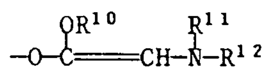

entsprechen. $R^{10}$ kann z.B. Benzyl, Phenyl oder $C_1$-$C_8$-Alkyl sein. $R^{11}$ und $R^{12}$ kann $C_1$-$C_8$-Alkyl, Phenyl oder Benzyl sein, oder sie können zusammen mit dem N-Atom einen gegebenenfalls weitere Heteroatome aus der Gruppe O, S oder N enthaltenden Heterocyclus bilden. $R^{10}$ und $R^{11}$ zusammen kann auch Methylen oder Ethylen darstellen. $R^{11}$ und $R^{12}$ können auch abspaltbare Schutzgruppen sein, z.B. $R^5_3$ Si- oder -$SiR^5_2$ $CH_2CH_2SiR^5_2$ -. $R^{10}$, $R^{11}$ und $R^{12}$ können wie zuvor für $R^1$ definiert substituiert sein. Eine andere bevorzugte Gruppe sind Enolreste der Formel

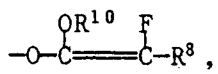

worin $R^8$ und $R^{10}$ die zuvor angegebene Bedeutung haben.

Bei $R^1$ kann es sich auch um den Rest eines Enamins oder Enhydrazin handeln, die über ein N-Atom an das Metall Me gebunden sind. Es kann sich um lineare oder cyclische Enamine bzw. Enhydrazine handeln, die bevorzugt bis zu 20, besonders 2-16 C-Atome enthalten. Die cyclischen Enamine und Enhydrazine können 3-8, besonders 4-6 Ringatome enthalten, wobei der Ring aus Atomen der Gruppe C, O, S und N gebildet sein kann. Das N-Atom der Enamingruppe kann substituiert sein, bevorzugt mit $C_1$-$C_8$-Alkyl, Phenyl, Benzyl oder mit Schutzgruppen wie z.B. $R^5_3$ Si-.

Der Enaminrest bzw. Enhydrazinrest kann z.B. der Formel

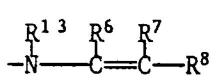

entsprechen, worin $R^6$, $R^7$ und $R^8$ die zuvor für den Enolrest $R^1$ angegebene Bedeutung haben, $R^6$ und $R^{13}$ zusammen mit der C-N-Gruppe oder $R^7$ und $R^{13}$ zusammen mit der -N-C=C-Gruppe einen 3-8-, vorzugsweise 4-6-gliedrigen Heterocyclus bilden, der weitere Heteroatome aus der Gruppe 0, S und N enthalten

kann, und $R^{13}$ H, $C_1$-$C_8$-Alkyl, Phenyl, Benzyl, $C_1$-$C_8$-Alkoxy, Phenoxy, Benzyloxy, $R^5_3$ Si- oder $-NR^{14}R^{15}$ bedeutet, worin $R^5$ die zuvor angegebene Bedeutung hat und $R^{14}$ und $R^{15}$ H, $C_1$-$C_8$-Alkyl, Phenyl oder Benzyl sind. In einer bevorzugten Gruppe sind $R^6$ H, $C_1$-$C_8$-Alkyl, Phenyl oder Benzyl und $R^7$ und $R^8$ H. Die Enol-, Enamin- und Enhydrazinreste sind C-Nukleophile, die zu Aldolreaktionen befähigt sind.

$R^1$ als Rest eines Enols kann auch ein Keton-, Ester- oder Amidenolat sein und enthält besonders bis zu 16, insbesondere 4-16 C-Atome. Die Enolate können sich ableiten von $\beta$-Ketoaldehyden, 1,3-Diketonen, $\beta$-Ketocarbonsäureestern und $\beta$-Ketocarbonsäureamiden. Die Estergruppe enthält bevorzugt Reste von aliphatischen $C_1$-$C_6$-Alkoholen. Das N-Atom in der Amidgruppe kann 1 oder 2-fach substituiert sein, bevorzugt mit $C_1$-$C_6$-Alkyl. Beispiele sind: $\beta$-Ketobutyraldehyd, Acetylaceton, Benzoylaceton, Acetylessigsäureethylester und Acetylessigsäureamid.

Der Rest $R^1$ kann ein- oder mehrfach, bevorzugt ein- bis dreifach, besonders ein- oder zweifach substituiert sein. Ist der Substituent Alkoxy oder Alkylthio, so enthält dieser bevorzugt 1-6 C-Atome. $R^4$ ist bevorzugt der Rest eines aliphatischen Alkohols mit 1 bis 6 C-Atomen. Es kann sich z.B. um Reste aliphatischer oder cycloaliphatischer Alkohole handeln. Beispiele sind Methoxy, Ethoxy, n-und i-Propoxy, n-, i-und t-Butoxy, Pentoxy, Hexyloxy, Cyclopentyloxy und Cyclohexyloxy.

$R^5$ enthält als Alkyl bevorzugt 1-6, besonders 1-4 C-Atome. Das Alkoxy, Alkylthio, -S-$C_2$-$C_4$-Alkylen-S- und -O-$C_2$-$C_4$-Alkylen-O-können an ein C-atom gebunden sein; es handelt sich dann um Acetal-oder Ketalgruppen.

Das Sekundäramino in der Bedeutung als Substituent kann der Formel $-NR^{16}R^{17}$ entsprechen, worin $R^{16}$ und $R^{17}$ $C_1$-$C_{12}$-, bevorzugt $C_1$-$C_6$-Alkyl, Cyclopentyl, Cyclohexyl, Phenyl, Benzyl oder $R^5_3$ Si-sind, oder $R^{16}$ und $R^{17}$ zusammen $C_3$-$C_6$-Alkylen, 3-Oxapentylen oder $-SiR^5_2$ -$C_2$-$C_3$-Alkylen-$SiR^5_2$ - bedeuten und $R^5$ die zuvor angegebene Bedeutung hat.

In einer bevorzugten Untergruppe ist $R^1$ gegebenenfalls ein- oder mehrfach durch Sekundäramino, Cyano, Nitro, $C_1$-$C_6$-Alkylthio, $C_1$-$C_6$-Alkoxy oder $-COR^4$, worin $R^4$ $C_1$-$C_{12}$-Alkoxy ist, substituiertes lineares oder verzweigtes $C_1$-$C_{18}$-Alkyl, $C_{-2}$-$C_{12}$-Alkenyl, $C_2$-$C_{12}$-Alkinyl, Cycloalkyl mit 3-8 Ring-C-Atomen, Cycloalkenyl mit 3 bis 8 Ring-C-Atomen, $C_6$-$C_{12}$-Aryl, $C_7$-$C_{16}$-Alkaryl oder Aralkyl, $C_8$-$C_{16}$-Alkaralkyl, $C_8$-$C_{16}$-Aralkenyl, $C_9$-$C_{16}$-Alkaralkenyl, $C_8$-$C_{16}$-Aralkinyl, $C_9$-$C_{16}$-Alkaralkinyl; oder ein über das Enolsauerstoffatom oder über das Enaminstickstoffatom gebundener Rest eine Enols, Enamins oder Enhydrazins.

In einer weiteren bevorzugten Untergruppe ist $R^1$ gegebenenfalls ein-oder mehrfach durch Sekundäramino, Cyano, Nitro, $C_1$-$C_6$-Alkoxy, $C_1$-$C_6$-Alkylthio oder $-COR^4$, worin $R^4$ $C_1$-$C_{12}$-Alkoxy ist, substituiertes lineares oder verzweigtes $C_1$-$C_6$-Alkyl, $C_2$-$C_6$-Alkenyl, $C_2$-$C_6$-Alkinyl, Cycloalkyl mit 3-6 Ring-C-Atomen, Cycloalkenyl mit 3 bis 6 Ring-C-Atomen, Phenyl, ($C_1$-$C_{10}$-Alkyl)phenyl, Phenyl($C_1$-$C_2$-alkyl), ($C_1$-$C_8$-Alkyl)-phenyl($C_1$-$C_2$-alkyl), Phenylvinyl, Phenylethinyl oder Phenylpropargyl, ($C_1$-$C_8$-Alkyl)phenylvinyl, ($C_1$-$C_8$-Alkyl)phenylethinyl oder ($C_1$-$C_7$-Alkyl)phenylpropargyl; oder ein über ein Enolsauerstoffatom oder Enaminstickstoffatom gebundener Rest eines Enols, Enamins oder Enhydrazins mit bis zu 20 C-Atomen.

Besonders bevorzugt ist $R^1$ gegebenenfalls ein- oder mehrfach durch Sekundäramino, Cyano, Nitro, $C_1$-$C_6$-Alkoxy, $C_1$-$C_6$-Alkylthio oder $-COR^4$, worin $R^4$ $C_1$-$C_{12}$-Alkoxy ist, substituiertes lineares oder verzweigtes $C_1$-$C_4$-Alkyl, Vinyl, Allyl, Ethinyl, Propargyl, Cyclopentyl, Cyclohexyl, Cyclopentenyl und Cyclohexenyl, Phenyl, Methylphenyl, Benzyl, 1-Phenyleth-2-yl, Methylbenzyl, Phenylvinyl, Methylphenylvinyl, Phenylethinyl, Phenylpropargyl, Methylphenylethinyl, Dimethylphenylethinyl oder -propargyl; oder ein über das Enolsauerstoffatom oder über das Enaminostickstoffatom gebundener Rest eines Enols, Enamins oder Enhydrazins mit 2-16 C-Atomen.

$R^2$ in der Bedeutung von Cyclopentadienyl, Alkoxy, Cycloalkoxy, Cycloalkylalkoxy, Aryloxy, Aralkyloxy, Alkylthio, Arylthio und Aralkylthio kann substituiert sein, beispielsweise mit 1 bis 3, bevorzugt 1 oder 2 Substituenten. Geeignete Substituenten sind zum Beispiel Alkyl mit bevorzugt 1 bis 6 C-Atomen, Alkenyl mit bevorzugt 2 bis 6 C-Atomen, Alkoxy mit bevorzugt 1 bis 6 C-Atomen, Cycloalkyl mit bevorzugt 5 oder 6- Ring-C-Atomen, Aryl mit bevorzugt 6 bis 12 C-Atomen, Aralkyl mit bevorzugt 7 bis 13 C-Atomen, Trialkoxysilyl mit bevorzugt 1 bis 6 C-Atomen in den Alkoxylgruppen, Trialkylsilyl mit bevorzugt 1 bis 6 C-Atomen in Alkylgruppen oder Halogen, bevorzugt F, Cl und Br. Aryl ist besonders Phenyl, und Aralkyl ist besonders Benzyl.

$R^2$ enthält als Alkoxy bevorzugt 1 bis 18, besonders 1 bis 12 C-Atomen. Beispiele sind: Methoxy, Ethoxy, n- oder i-Propoxy, n-, i-oder t-Butoxy, Pentoxy, Hexoxy, 2-Ethylhexoxy, Octoxy, Nonoxy, Decoxy, Undecoxy, Dodecoxy, Tri-t-butylmethoxy, Tetradecoxy, Octadecoxy. $R^2$ als Cycloalkoxy und Cycloalkylalkoxy enthält bevorzugt 4-8, besonders 5 oder 6 Ring-C-Atome und insgesamt vorzugsweise 4-18, besonders 4-12 C-Atome. Beispiele sind Cyclopentoxy, Cyclohexoxy, Cyclohexylmethyloxy, Triptycen-9-oxy.

$R^2$ ist als Aryloxy besonders $C_6$-$C_{12}$-Aryloxy und bevorzugt Napthoxy oder Phenoxy; und als Aralkoxy besonders $C_7$-$C_{12}$-Aralkyloxy, bevorzugt Benzyloxy oder Phenylethoxy.

$R^2$ enthält als Alkylthio bevorzugt 1-18, besonders 1-12 C-Atome. Das Alkylthio kann linear oder verzweigt sein. Beispiele sind Methylthio, Ethylthio, n- oder i-Propylthio, n-, i- oder t-Butylthio, Pentylthio, Hexylthio, Octylthio, Dodecylthio, Octadecylthio.

$R^2$ als Arylthio weist insbesondere 6-12 C-Atome auf und ist bevorzugt Phenylthio oder Naphthylthio; und als Aralkylthio weist $R^2$ besonders 7-16 C-Atome auf und ist bevorzugt Benzylthio oder Phenylethylthio.

$R^2$ kann als Halogen F, Cl, Br oder J und als Pseudohalogen CN, CNS oder CNO sein.

Als Acyloxy und Acylamino enthält $R^2$ bevorzugt 1 bis 18, besonders 1 bis 12 C-Atome. Das N-Atom im Acylamino kann mit $C_1$-$C_6$-Alkyl substituiert sein. Das Acyloxy und Acylamino kann sich z.B. von aliphatischen $C_1$-$C_{18}$-, bevorzugt $C_1$-$C_{12}$-Carbonsäuren, aromatischen $C_7$-$C_{13}$-Carbonsäuren oder araliphatischen $C_8$-$C_{14}$-Carbonsäuren ableiten. Beispiele für Säuren, von denen sich Acyloxy und Acylamin ableiten kann sind: Ameisensäure, Essigsäure, Chloressigsäure, Trichloressigsäure, Trifluoressigsäure, Propionsäure, Buttersäure, Benzoesäure, Chlorbenzoesäure, Phenylessigsäure.

$R^2$ in der Bedeutung von Trialkylsilyloxy enthält bevorzugt 1 bis 6 C-Atome in den Alkylgruppen. Beispiele für Alkylgruppen sind Methyl, Ethyl, n- oder i-Propyl, n-, i- oder t-Butyl, Pentyl und Hexyl. Beispiele für Trialkylsilyloxy sind Trimethyl-, Triethyl-, Triisopropyl-, Tri-n-Butyl- und Tri-t-Butylsilyloxy.

In einer bevorzugten Ausführungsform bedeutet $R^2$ gegebenenfalls durch $C_1$-$C_6$-Alkyl, $C_2$-$C_6$-Alkenyl, $C_1$-$C_6$-Alkoxy, Cycloalkyl mit 5 oder 6 Ring-C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, oder Br substituiertes Cyclopentadienyl, $C_1$-$C_{18}$-Alkoxy, $C_6$-$C_{12}$-Aryloxy, $C_7$-$C_{16}$-Aralkyloxy, $C_1$-$C_{18}$-Alkylthio, $C_6$-$C_{12}$-Arylthio, $C_7$-$C_{16}$-Aralkylthio; Halogen, Pseudohalogen, $C_1$-$C_{18}$-Acyloxy, $C_1$-$C_{18}$-Acylamino oder Trialkylsilyloxy mit 1 bis 6 C-Atomen in den Alkylgruppen.

In einer anderen bevorzugten Ausführungsform bedeutet $R^2$ gegebenenfalls durch $C_1$-$C_6$-Alkyl, $C_2$-$C_6$-Alkenyl, $C_1$-$C_6$-Alkoxy, Cycloalkyl mit 5 oder 6 Ring-C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, oder Br substituiertes Cyclopentadienyl, $C_1$-$C_6$-Alkoxy, Phenoxy, Benzyloxy, $C_1$-$C_6$-Alkylthio, Phenylthio, Benzylthio; Cl, Br, J, CN, CNS, CNO, $C_1$-$C_{12}$-Acyloxy, $C_1$-$C_{12}$-Acylamino oder Trialkylsilyloxy mit 1 bis 4 C-Atomen in den Alkylgruppen.

Besonders bevorzugt ist $R^2$ Cyclopentadienyl oder Methylcyclopentadienyl.

$R^3$ bedeutet beovzugt den Rest eines geschützten monohydroxy-, monothiol- oder monoaminofunktionellen $C_3$-$C_7$-Monosaccharids oder entsprechender Disaccharide oder Trisaccharide, oder deren Derivate aus der Gruppe der Zuckeralkohole, Ester einer Zuckersäure, Aldozuckersäure oder Ketozuckersäure, Aminozucker, Desoxyzucker oder Zuckermercaptale. Besonders bevorzugt ist $R^3$ der Rest eines $C_3$-$C_7$-, besonders $C_5$- oder $C_6$-Monosaccharids oder deren Derivate. Insbesondere ist $R^3$ der Rest einer geschützten Furanose oder Pyranose.

Die Hydroxylgruppen der Zucker und von deren Derivaten sind geschützt. Als geeignete Schutzgruppen kommen $C_1$-$C_8$-Acyl, $C_1$-$C_8$-Alkyl, Benzyl, Diphenylmethyl, Trityl, $C_1$-$C_8$-Alkyliden, $C_3$-$C_8$-Cycloalkyliden, Triphenylsilyl oder Trialkylsilyl mit 1 bis 8 C-Atomen in den Alkylgruppen sowie Diphenyl Si= oder ($C_1$-$C_8$-Alkyl)Si= und DiphenylSn= oder ($C_1$-$C_8$-Alkyl)$_2$Sn= in Frage. Das Alkyliden kann z.B. durch Phenyl substituiert sein. Das Acyl enthält bevorzugt 2 bis 7 C-Atome, das Alkyl bevorzugt 1 bis 4 C-Atome, die Alkylgruppen im Trialkylsilyl bevorzugt 1 bis 6 C-Atome, das Alkyliden bevorzugt 2 bis 6 C-Atome und das Cycloalkyliden bevorzugt 5 oder 6 Ring-C-Atome. Beispiele sind Methyl, Ethyl, n- oder i-Propyl, n-, i- oder t-Butyl, Formyl, Acetyl, Propionyl, Pivaloyl, Benzoyl, Methyliden, Ethyliden, 1,1- oder 2,2-Propyliden, 1,1- oder 2,2-Butyliden, Benzyliden, Pentyliden, Hexyliden, Cyclohexyliden, Trimethylsilyl, Dimethyl-t-butylsilyl, (1,1,2,2-Tetramethylethyl)dimethylsilyl, (t-Butyl)$_2$Si=, $(CH_3)_2$Si, (i-Propyl)$_2$Si=, (n-Butyl)$_2$Sn=. Weitere, geeignete Schutzgruppen sind $C_1$-$C_8$-Alkoxy- oder Phenoxy B$\langle$ oder Phenyl-B$\langle$ sowie Alkoxymethyl, z.B. Methoxymethyl, Ethoxymethyl oder (Trimethylsilylethyloxy)methyl.

Bei den Zuckern, von denen sich $R^3$ ableitet, kann es sich zum Beispiel um Aldosen oder Ketosen handeln. Beispiele sind Glycerinaldehyd, Erythose, Threose, Arabinose, Ribose, Xylose, Lyxose, Glucose, Mannose, Allose, Galactose, Fructose, Gulose, Altrose, Idose, Talose, Ribulose, Erythrulose, Xylulose, Psicose, Sorbose, Tagatose. Die Zucker können offenkettig oder cyclisiert z.B. als Furanosen oder Pyranosen vorliegen.

Beispiele für Di- und Trisaccharide sind Sucrose, Maltose, Lactose, Raffinose.

Beispiele für Zuckeralkohole, von denen sich $R^3$ ableiten kann, sind Sorbit und Mannit.

Die Zuckersäuren, Aldo- und Ketozuckersäuren, von denen sich $R^3$ ableiten kann, können als Lactone oder Ester vorliegen, wobei die Estergruppe oder Estergruppen vorzugsweise $C_1$-$C_4$-Alkyl, insbesondere Methyl enthalten. Beispiele für solche Säuren sind Gluconsäure, Zuckersäure, Mannozuckersäure, Mannonsäure und Uronsäuren wie z.B. Glucuronsäure, sowie Neuraminsäure und Ascorbinsäure.

Als Beispiele für Aminozucker, von denen sich $R^3$ ableiten kann, seien Glucosamin, Galactosamin und Mannosamin genannt.

Als Beispiele für Zuckermercaptale, von dem sich $R^3$ ableiten kann, seien Glucosedimethylmercaptal und Thioglucosid genannt.

Beispiele für Desoxyzucker von denen sich $R^3$ ableiten kann, sind 2-Desoxyribofuranose, Rhamnose, Fucose und Digitoxose.

Die Zucker und Zuckerderivate, von denen sich $R^3$ ableitet, liegen als praktisch reine Enantiomere vor.

Ein bevorzugtes Beispiel eines geschützten, monohydroxyfunktionellen Zuckers, von dem sich der Rest $R^3$ ableiten kann, ist 1,2:5,6-Di-O-isopropyliden-α-D-glucofuranose.

In Formel I steht x bevorzugt für 1 und in Formel Ia steht y bevorzugt für 2, besonders für 1.

In der Gruppe $M^{\oplus}$ der Formel Ia steht Y bevorzugt für Cl, Br oder J. $M^{\oplus}$ bedeutet bevorzugt $Li^{\oplus}$, $MgCl^{\oplus}$, $MgBr^{\oplus}$, $ZnCl^{\oplus}$, $ZnBr^{\oplus}$, $CdCl^{\oplus}$, $CdBr^{\oplus}$ oder Tetraalkylammonium mit 1 bis 6 C-Atomen in den Alkylgruppen. Besonders bevorzugt bedeutet $M^{\oplus}$ $MgY^{\oplus}$, worin Y Cl, Br oder J ist.

Die Verbindungen der Formeln I und Ia können z.B. hergestellt werden, indem man eine Verbindung der Formel II

$$R^2_x R^3_{3-x} MeX \qquad (II)$$

oder eine Verbindung der Formel (IIa)

$$R^2_y R^3_{4-y} Me \qquad (IIa)$$

in Gegenwart eines inerten Lösungsmittels und eines inerten Schutzgases mit einer Verbindung der Formel III

$R^{1\ominus} M^{\oplus}$ (III)

umsetzt, wobei Me, $R^1$, $R^2$, $R^3$, $M^{\oplus}$, x und y die zuvor angegebene Bedeutung haben und X für ein Anion steht. Als Anion kommen z.B. $PF_6^{\ominus}$,$SbF_6^{\ominus}$, $BF_4^{\ominus}$, $CF_3COO^{\ominus}$, Sulfonat (z.B. Tosylat), und besonders $Cl^{\ominus}$ oder $Br^{\ominus}$ in Frage.

Die Verbindungen der Formel II sind ein weiterer Gegenstand der Erfindung, sie sind in einfacher Weise durch die Umsetzung von 1 Mol eines Salzes der Formel

$R^2_x MeX_{4-x}$

mit 1 bis 3 Mol eines optisch aktiven, geschützten, monohydroxy-, monothiol- oder monoaminofunktionellen, Zuckers, Thiozuckers, Aminozuckers oder deren Derivate aus der Gruppe der Zuckeralkohole; Ester einer Zucker-, Aldo- oder Ketozuckersäure; Aminozucker, Zuckermercaptale oder Desoxyzucker der Formel $R^3H$ erhältlich. Die Umsetzung kann bei Temperaturen von -78 bis +100 °C vorzugsweise in einem inerten Lösungsmittel, wie z.B. Ethern (Diethylether, Tetrahydrofuran, Dioxan, Ethylenglykoldimethylether) erfolgen. Zweckmässig wird die Reaktion unter Schutzgasatmosphäre, zum Beispiel Argon, und in Gegenwart einer basischen Verbindung durchgeführt, um das entstehende HX zu binden. Geeignete basische Verbindungen sind zum Beispiel Alkalicarbonate, wie Natriumcarbonat und Natriumbicarbonat sowie Amine, besonders tertiäre Amine, wie z.B. Triethylamin. Zur Isolierung der Verbindungen wird das ausgefallene Halogenid abfiltriert und das Lösungsmittel entfernt. Die so erhaltenen Rohprodukte können ohne Reinigung weiterverwendet werden. Die Titanhalogenide sind bekannt oder können nach bekannten Verfahren hergestellt werden. Anstelle der Me-Halogenide können auch Verbindungen der Formel $R^4_4$ Me eingesetzt werden, wobei $R^1$ insbesondere Alkyl ist. Ferner können auch Verbindungen der Formel $R^1_z$ $TiZ_{4-z}$ verwendet werden, worin Z Cl oder Br bedeutet und z 1, 2 oder 3 ist. Wenn hierbei 3 Mol Zucker eingesetzt werden, gelangt man drekt zu erfindungsgemässen Verbindungen der Formel I vom Typ $R^1MeR^3_3$ .

Die Herstellung der Verbindungen der Formel I und Ia erfolgt zweckmässig bei Temperaturen von -78 bis +30 °C und in einem inerten Lösungsmittel. Geeignete Lösungsmittel sind insbesondere Ether. Die Reaktion wird unter Schutzgas, z.B. Argon durchgeführt. Ausgefallenes $M^{\oplus}X^{\ominus}$ wird abfiltriert. Das nach Entfernen des Lösungsmittels erhaltene Rohprodukt kann direkt weiterverwendet werden.

Die erfindungsgemässen Verbindungen eignen sich hervorragend als enantioselektive Reaktanden für Verbindungen mit Carbonyl- und/oder N-substituierten Imingruppen. Wenn $R^2$ einen Cyclopentadienylrest bedeutet, eignen sich die Verbindungen insbesondere zur Uebertragung von Allyl-, Enol-, Enamin- und Enhydrazingruppen. Wenn $R^2$ nicht ein Cyclopentadienylrest ist, eignen sich die Verbindungen zur Uebertragung von allen $R^1$-Gruppen. Ein weiterer Gegenstand der Erfindung ist diese Verwendung. Bei den Carbonylverbindungen handelt es vorzugsweise um prochirale Aldehyde und Ketone; als Reaktionsprodukte erhält man in hohen Ausbeuten chirale sekundäre oder tertiäre Alkohole bzw. sekundäre Amine oder Aminosäuren mit hohem Ueberschuss eines Enantiomeren. Die Synthese von chiralen Wirkstoffen im Bereich der Pharmaka und Agrarchemikalien hat grosse Bedeutung erlangt. Die erfindungsgemässen Verbindungen eignen sich zur Herstellung von entsprechenden Zwischenprodukten für die Synthese solcher Wirkstoffe oder um in der Endstufe der Synthese solcher Wirkstoffe Gruppen mit chiralen C-Atomen einzuführen. So können z.B. in hohen Ausbeuten Pheromone für die Insektenbekämpfung hergestellt werden. Als Beispiel sei (-)-S-Ipsenol genannt, dessen Synthese in den Beispielen beschrieben wird.

Die erfindungsgemässen Verbindungen sind darüber hinaus preisgünstig aus billigen Ausgangsstoffen herstellbar. Als Derivate von natürlichen Verbindungen können sie mittels biologischer Abbaumethoden umweltfreundlich entsorgt werden, zumal Titan bzw. Titanoxid bekannterweise physiologisch unbedenklich ist. Zucker und Zuckerderivate sind in grosser Vielfalt bekannt, so dass auch die Möglichkeit besteht, Reaktanden mit hoher Wirksamkeit für bestimmte Carbonylgruppen bzw. N-substituierte Imingruppen bereitzustellen. Bei dem Substituenten im Imin kann es sich um die zuvor erwähnten Reste wie Alkyl, Alkenyl, Cycloalkyl, Aryl und Aralkyl handeln. Sind diese Reste durch Carboxyl oder Carboxylestergruppen substituiert, erhält man als Reaktionsprodukte Aminosäuren bzw. deren Ester.

Ein weiterer Gegenstand vorliegender Erfindung ist ein Verfahren zur Herstellung von sekundären und tertiären Alkoholen und sekundären Aminen, das dadurch gekennzeichnet ist, dass man Aldehyde, Ketone, N-substituierte Aldimine oder Ketimine mit 1 Mol einer Verbindung der Formel I oder Ia pro Mol Aldehyd-, Keto- oder Imingruppe umsetzt.

Die Reaktion wird vorteilhaft bei Temperaturen von -80 bis 30 °C in Gegenwart eines inerten Lösungsmittels und unter Schutzgas durchgeführt. Zur Isolierung des Reaktionsproduktes wird zweckmässig hydrolysiert, das Produkt extrahiert und in üblicher Weise gereinigt. Geeignete inerte Lösungsmittel sind beispielsweise Ether oder Kohlenwasserstoffe, wie Pentan, Hexan, Cyclohexan, Methylcyclohexan, Benzol, Toluol, Xylol, Diethylether, Tetrahydrofuran, Dioxan, Nitrile wie z.B. Acetonitril, oder chlorierte Kohlenwasserstoffe wie z.B. Methylenchlorid, Chloroform oder Chlorbenzol.

Die nachfolgenden Beispiele erläutern die Erfindung. Cp steht für Cyclopentadienyl, HODAG für den Zucker gemäss Beispiel 1a (Abkürzung für Diacetonglucose). Die Bestimmung des Enantiomerenüberschusses erfolgt nach folgenden Methoden:

A. Polarimetrie
Referenz:
(S)-1-Phenyl-3-buten-1-ol $(\alpha)_D$ = 48,7° (c 6,92, Benzol)
A.F. Lee, Holding and W.A. Ross, J. Chem. Arc. 1954, 145.
B. GC-Analyse (DB-Wax 30 m) der MTP A-Ester
Referenz:
James A. Date, David L. Dull and Harry S. Mosher, JOC 34, 2543 (1969).
C. GC-Analyse (Chirasil-L-val 50 M) der Derivate mit Isopropylisocyanat.
Referenz:
W. König et al., Journal of Chromatography, 239 (1982) 227-231.
D. $^1$H-NMR-Shift-Verfahren mit optisch aktivem 1-(9′-Anthryl)-2,2,2-trifluorethanol.
E. Wie B, aber mit Chirasil-L-val, 50 m.
F. HPLC-Analyse (Säule mit Cellulosetriacetat).
G. Wie C, aber ohne Derivatisierung.
H. HPLC-Analyse (R-Ion DNBPG).
I. Derivatisierung mit Trifluoressigsäureanhydrid, dann wie C.

Beispiel 1

a) Cyclopentadienylchlorotitanium-bis-(1,2:5,6-Di-O-isopropyliden-α-glucofuranose)-Komplex

Zu einer Lösung von 2,15 g (9,8 mmol) Cyclopentadienyltitantrichlorid und 5,10 g (19,6 mmol) 1,2:5,6-Di-O-isopropyliden-α-glucofuranose in 60 ml Diethylether (destilliert über Natrium und Benzophenon) wird innert 30 Minuten bei Raumtemperatur (RT) eine Lösung von 2,03 g (20,1 mmol) Triethylamin in 25 ml

Diethylether abs. zugetropft. Die hellgelbe Suspension wird bei RT für weitere 2 Stunden gerührt, das gebildete Triethylaminhydrochlorid wird unter Argon abgenutscht und das Lösungsmittel am Vakuum abdestilliert. Das erhaltene ölige, schwach gelbliche Rohprodukt kann direkt weiterverwendet werden. $^1$H-NMR-Spektrum ($CDCl_3$, 60 MHz): 1,31-1,49 (m, 24 H), 3,73-4,60 (m, 10 H), 5,07 (d, 2 Hz, 2 H), 5,83 (d, 4 Hz, 2 H), 6,62 (s, 5 H).

b) Cyclopentadienylallyltitanium-bis-(1,2:5,6-Di-O-isopropyliden-α-glucofuranose)-Komplex

9,8 mmol Cyclopentadienylchlorotitanium-bis-(1,2:5,6-Di-O-isopropyliden-α-glucofuranose)-Komplex werden in 100 ml Diethylether gelöst, auf 0 °C abgekühlt und mit 3,8 ml (9,8 mmol) Allylmagnesiumchlorid (2,6 molare Lösung in Tetrahydrofuran) versetzt. Die orange-rote Suspension wird 1 Stunde bei 0 °C gerührt, unter Argon filtriert und im Vakuum eingedampft. Das luftempfindliche ölige Rohprodukt wird direkt weiterverwendet. $^1$H-NMR-Spektrum ($CDCl_3$, 60 MHz): 1,32-1,49 (m, 24 H), 3,23-4,53 (m, 15 H), 4,85 (d, 2 Hz, 2 H), 5,83 (d, 4 Hz, 2 H), 6,27 (s, 5 H).

Beispiel 2:

a) Cyclopentadienyltitanium-tris-(1,2:5,6 -Di-O-isopropyliden-α-glucofuranose)-Komplex

Zu einer Lösung von 2,19 g (10 mmol) Cyclopentadienyltitantrichlorid und 7,80 g (30 mmol) 1,2:5,6-Di-O-isopropyliden-α-glucofuranose in 60 ml Diethylether wird innert 30 Minuten bei RT eine Lösung von 3,03 g (30 mmol) Triethylamin in 30 ml Diethylether zugetropft. Die schwach gelbe Suspension wird bei RT für weitere 2 Stunden gerührt, das gebildete Triethylaminhydrochlorid wird unter Argon abgenutscht und das Lösungsmittel am Vakuum abdestilliert. Das erhaltene schwach gelbliche Rohprodukt erstarrt bei RT und kann direkt weiterverwendet werden. $^1$H-NMR-Spektrum ($CDCl_3$, 60 MHz): 1,28-1,47 (m, 36 H), 3,80-4,50 (m, 15 H), 4,83 (d, 2 Hz, 3 H), 5,73 (d, 4 Hz, 3 H), 6,48 (s, 5 H).

b) Cyclopentadienyltitanium-tris-(1,2:5,6-Di-O-isopropyliden-α-glucofuranose)-allylmagnesiumchlorid-Komplex

10 mmol Cyclopentadienyltitanium-tris(1,2:5,6-Di-O-isopropyliden-α-glucofuranose)-Komplex werden in 100 ml Diethylether gelöst, auf 0 °C abgekühlt und mit 3,85 ml (10 mmol) Allylmagnesiumchlorid (2,6 molare Lösung in Tetrahydrofuran) versetzt. Die orange-rote Lösung wird eine Stunde bei 0 °C weitergerührt und dann direkt weiterverwendet. $^1$H-NMR-Spektrum ($CDCl_3$, 60 MHz): 1,30-1,48 (m, 36 H), 3,50-4,57 (m, 20 H), 4,86 (d, 2 Hz, 3 H), 5,76 (d, 4 Hz, 3 H), 6,50 (s, 5 H).

Anwendungsbeispiele

Beispiel 3:

Herstellung von (R)-1-Phenyl-3-buten-1-ol

9,8 mmol Cyclopentadienylallyltitanium-bis(1,2:5,6-Di-O-isopropyliden-α-glucofuranose)-Komplex gemäss Beispiel 1, gelöst in 100 ml Diethylether, wird mit einem $CO_2$/Aceton-Bad auf -74 °C abgekühlt und mit 0,89 ml (8,8 mmol) Benzaldehyd (frisch destilliert) versetzt. Die gelbe, klare Lösung wird 2 Stunden bei -74 °C weitergerührt und mit 50 ml $NH_4F$-Lösung (45%ig in Wasser) versetzt.

Die beige Emulsion wird auf RT erwärmt und im Scheidetrichter getrennt. Die Etherphase wird 1x mit 50 ml und 2x mit 30 ml 2N HCl extrahiert, dann mit 300 ml Ether verdünnt, anschliessend 2x mit je 300 ml Wasser und 1x mit 300 ml NaCl-Lösung (gesättigt) gewaschen, mit $MgSO_4$ getrocknet, filtriert und am Rotavapor zur Trockene eingedampft. Das Rohprodukt wird Flash-chromatographiert (45 g Kieselgel, 25 cm lang, ∅ 2 cm, 10 ml/Fraktion, 0,3 bar, Ether/n-Hexan 1:5) und destilliert (Kp 150 °C/14 mbar); man erhält 1,02 g (79 % Ausbeute) klares, farbloses Oel: $[\alpha]_D$ = + 44,1 ° (c 6,5, Benzol), 91 % ee (Methoden A und B).

Beispiel 4-27:

Analog zu Beispiel 3 werden die in nachfolgender Tabelle aufgeführten Reaktionen durchgeführt: LM = Lösungsmittel, T = Reaktionstemperatur.

Tabelle

| Bsp. | Carbonylverbindung | Titan-komplexe | Reaktionsbedingungen LM | T[°C] | Produkt | Ausbeute [%] | $[\alpha]_D$ | % ee | Methode |
|---|---|---|---|---|---|---|---|---|---|
| 4 | 8,8 mmol Benzaldehyd | 9,8 mmol gemäss Beispiel 1 | Toluol | -74° | 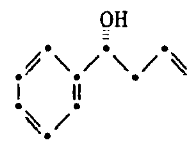 | 80 | + 43,62 (c 6,52; Benzol) | 90 | A B C |
| 5 | 9 mmol Benzaldehyd | 10 mmol gemäss Beispiel 2 | Diethylether | -74° | 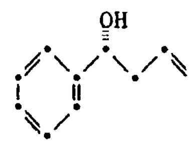 | 70 | + 35,6 (c 6,7; Benzol) | 73 71 | A C |
| 6 | 7,8 mmol Cyclohexylaldehyd | 9,8 mmol gemäss Beispiel 1 | Diethylether | -74° | 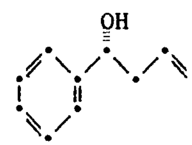 | 78 | + 8,2 (c 0,55; Ethanol) | 92 | B |
| 7 | 7 mmol 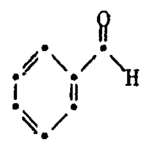 | 8 mmol gemäss Beispiel 1 | Diethylether | 0° | 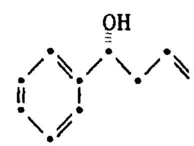 | 98 | (+) b) | 81 | C D |

Tabelle (Fortsetzung)

| Bsp. | Carbonylverbindung | Titan-komplexe | Reaktionsbedingungen LM | T[°C] | Produkt | Ausbeute [%] | $[\alpha]_D$ | % ee | Methode |
|---|---|---|---|---|---|---|---|---|---|
| 8 | 7 mmol 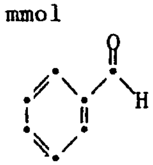 | 8 mmol gemäss Beispiel 1 | Tetrahydrofuran | -74° | 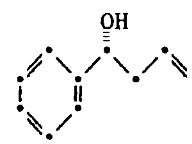 | 85 | (+) b) | 89 | C |
| 9 | 10 mmol 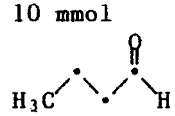 | 12 mmol gemäss Beispiel 1 | Diethylether | -74° | 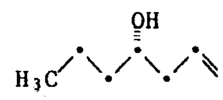 | 50 | (-) b) | 94 | E |
| 10 | 10 mmol 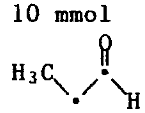 | 12 mmol gemäss Beispiel 1 | Diethylether | -74° | 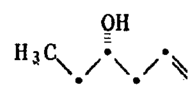 | 67 | - 8,5 (1,9, Benzol) | 94 | C |
| 11 | 36 mmol 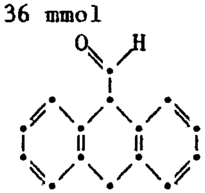 | 44 mmol gemäss Beispiel 1 | Diethylether | -74° | 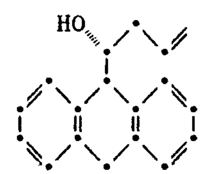 | 90 | + 19,9 (5,93, Benzol) | 94 | F |

Tabelle (Fortsetzung)

| Bsp. | Carbonylverbindung | Titan-komplexe | Reaktionsbedingungen LM | T[°C] | Produkt | Ausbeute [%] | $[\alpha]_D$ | % ee | Methode |
|---|---|---|---|---|---|---|---|---|---|
| 12 | 10 mmol 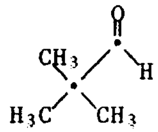 | 12 mmol gemäss Beispiel 1 | Diethylether | -74° | 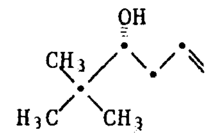 | 35 | + 10,86 (10,53 Benzol) | 92 | C |
| 13 | 10 mmol 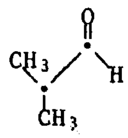 | 12 mmol gemäss Beispiel 1 | Diethylether | -74° | 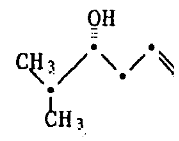 | 98 | (+) b) | 92 | C |
| 14 | 10 mmol 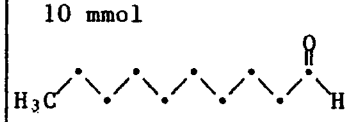 | 12 mmol gemäss Beispiel 1 | Diethylether | -74° | 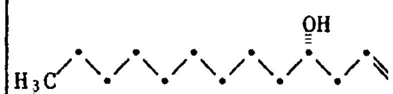 | 88 | + 10,38 (6,65, Benzol) | 92 | C |
| 15 | 10 mmol 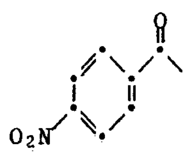 | 12 mmol gemäss Beispiel 1 | Diethylether | -74° | 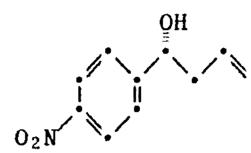 | 82 | + 23,7 (5,1, Benzol) | 92 | C |

Tabelle (Fortsetzung)

| Bsp. | Carbonylverbindung | Titan-komplexe | Reaktionsbedingungen LM | Reaktionsbedingungen T[°C] | Produkt | Ausbeute [%] | $[\alpha]_D$ | % ee | Methode |
|---|---|---|---|---|---|---|---|---|---|
| 16 | 10 mmol 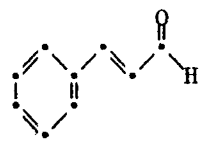 | 12 mmol gemäss Beispiel 1 | Diethylether | -74° | 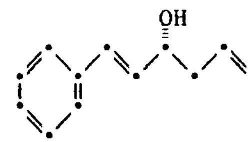 | 52 | + 13,5 (9,73, $Et_2O$) | 90 | A C |
| 17 | 10 mmol 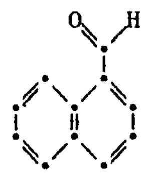 | 12 mmol gemäss Beispiel 1 | Diethylether | -74° | 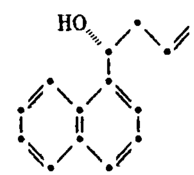 | 84 | + 77,2 (10,24 Benzol) | 88 | C |
| 18 | 10 mmol 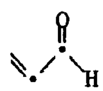 | 12 mmol gemäss Beispiel 1 | Diethylether | -74° | 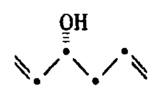 | 61 | (-) b) | 87 | E |
| 19 | 15 mmol 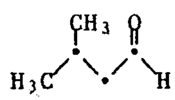 | 17,2 mmol gemäss Beispiel 1 | Diethylether | -74° | 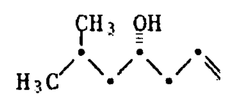 | 55 | (-) b) | 86 | C |

Tabelle (Fortsetzung)

| Bsp. | Carbonylverbindung | Titan-komplexe | Reaktionsbedingungen | | Produkt | Ausbeute [%] | $[\alpha]_D$ | % ee | Methode |
|---|---|---|---|---|---|---|---|---|---|
| | | | LM | T[°C] | | | | | |
| 20 | 8,8 mmol 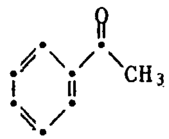 | 9,8 mmol gemäss Beispiel 1 | Diethyl-ether | 0° | 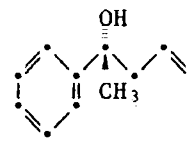 a) | 57 | + 22,7 (5,78, Benzol) | 76 | A D |
| 21 | 8,8 mmol 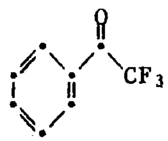 | 9,8 mmol gemäss Beispiel 1 | Diethyl-ether | 0° | 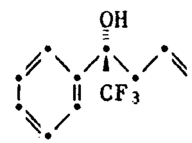 a) | 60 | - 35,3 (6,92, $CH_2Cl_2$) | 52 | A D |
| 22 | 7,8 mmol 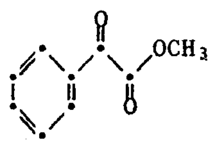 | 9,8 mmol gemäss Beispiel 1 | Diethyl-ether | 20° | 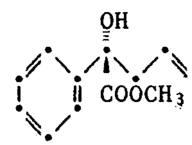 a) | 60 | + 10,69 (5,55, Benzol) | 52 | D |
| 23 | 8,8 mmol 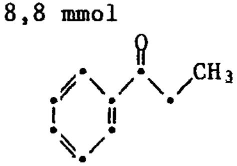 | 9,8 mmol gemäss Beispiel 1 | Diethyl-ether | 0° | 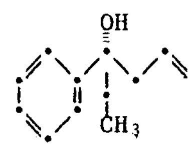 a) | 70 | (+) b) | 44 | D |

Tabelle (Fortsetzung)

| Bsp. | Carbonylverbindung | Titan-komplexe | Reaktionsbedingungen LM | Reaktionsbedingungen T[°C] | Produkt | Ausbeute [%] | [α]$_D$ | % ee | Methode |
|---|---|---|---|---|---|---|---|---|---|
| 24 | 8 mmol 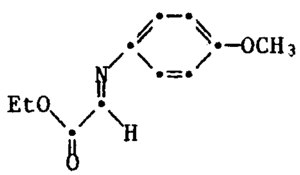 | 10 mmol gemäss Beispiel 1 | Diethylether | -20° | a) 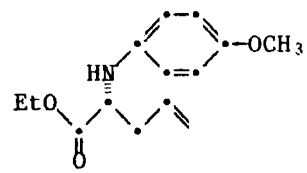 | 16 | b) | 37 | G |
| 25 | 5 mmol 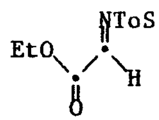 | 6,25 mmol gemäss Beispiel 1 | Diethylether | -74° | a) 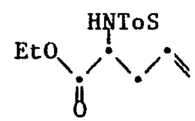 | 34 | b) | 26 | G |
| 26 | 8 mmol 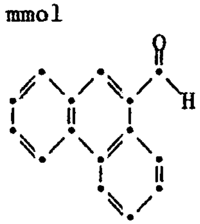 | 10 mmol gemäss Beispiel 1 | Diethylether | -74° | a) 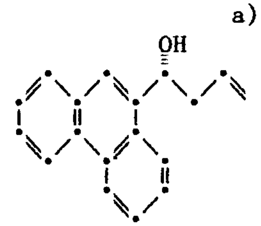 | 60 | b) | 92 | H |

ToS = Tosyl

Tabelle (Fortsetzung)

| Bsp. | Carbonylverbindung | Titan-komplexe | Reaktionsbedingungen LM | T[°C] | Produkt | Ausbeute [%] | $[\alpha]_D$ | % ee | Methode |
|---|---|---|---|---|---|---|---|---|---|
| 27 | 8 mmol 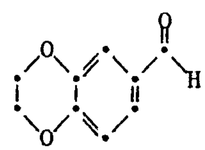 | 10 mmol gemäss Beispiel 1 | Diethylether | -74° | 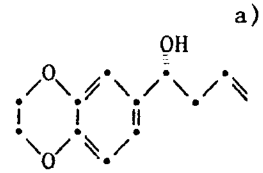 a) | 40 | b) | ≥95 | H |

a) absolute Konfiguration unbekannt

b) Drehwert nicht bestimmt

Beispiel 28-30:

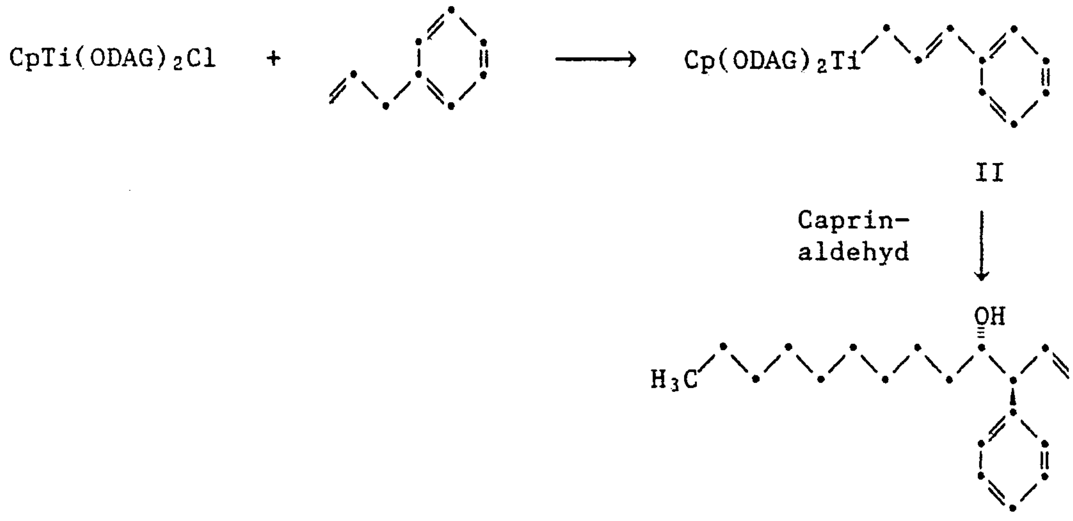


II

III

2,95 g (25 mmol) Allylbenzol wird in 170 ml absolutem Tetrahydrofuran bei -27°C mit 15,63 ml n-Butyllithium-Lösung (1,6 m in Hexan) umgesetzt und anschliessend bei RT 60 Minuten gerührt (Rotfärbung). Diese Lösung wird bei 0°C zu 25 mmol $CpTi(ODAG)_2Cl$, gelöst in 270 ml absolutem Ether, zugetropft und weitere 30 Minuten gerührt. Die resultierende Lösung von II wird auch für die Beispiele 29 und 30 verwendet (siehe Tabelle 2).

126 ml (8 mmol) Lösung II wird bei -78°C mit 1,24 ml (6,6 mmol) Caprinaldehyd versetzt und 2 Stunden gerührt. Die Lösung wird mit 30 ml 45%iger wässriger $NH_4F$-Lösung versetzt, filtriert und mit Ether extrahiert. Die organische Phase wird 2 x mit gesättigter NaCl-Lösung gewaschen, mit $MgSO_4$ getrocknet und eingedampft.

Die Chromatographie an Kieselgel (Hexan/Ether = 3:1) ergibt 1,02 g (3,7 mmol) Alkohol III (56 % Ausbeute); 86 % ee bestimmt nach Methode C. Die Reaktion verläuft mit 100 % anti-Selektivität.

Tabelle 2

| Bsp. | Carbonylverbindung | Titan-komplexe | Reaktionsbedingungen LM | Reaktionsbedingungen T[°C] | Produkt | Ausbeute [%] | % ee | Methode | Diastereo-Selektivität |
|---|---|---|---|---|---|---|---|---|---|
| 29 | 7 mmol 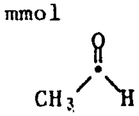 | 126 ml (8 mmol) Lösung II aus Beispiel 28 | Diethylether, Thf | -74° | 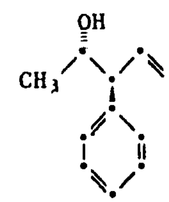 | 70 | 86 | C | 100 % anti |
| 30 | 7 mmol 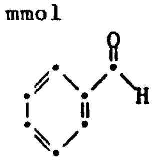 | 126 mmol (8 mmol) Lösung II aus Beispiel 28 | Diethylether, Thf | -74° | 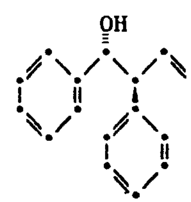 | 80 | 80 | C | 100 % anti |

Thf = Tetrahydrofuran

Beispiel 31:

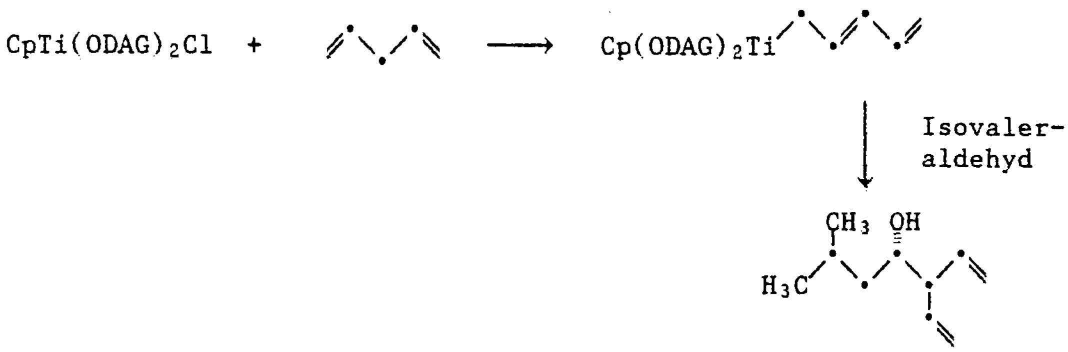


I

1,23 ml (12 mmol) 1,4-Pentadien wird in 20 ml absolutem Tetrahydrofuran bei -60 °C mit 6,25 ml n-Butyllithium-Lösung (1,6 m in Hexan) umgesetzt und anschliessend bei RT 30 Minuten gerührt. Diese Lösung wird bei 0 °C zu 100 mmol CpTi(ODAG)$_2$Cl, gelöst in 105 ml Diethylether (Ether), zugetropft und weiter 30 Minuten gerührt. Bei -78 °C wird 1,08 ml (10 mmol) Isovaleraldehyd zugegeben und 2 Stunden gerührt.

Die Lösung wird mit 30 ml 45%iger wässriger $NH_4F$-Lösung versetzt, filtriert und mit Ether extrahiert. Die organische Phase wird 2 x mit gesättigter NaCl-Lösung gewaschen, mit $MgSO_4$ getrocknet und eingedampft.

Das Rohprodukt wird in 100 ml 0,2 n HCl aufgeschlämmt und 1,5 Stunden bei Raumtemperatur (RT) gerührt. Die Wasserphase wird 4 x mit Ether extrahiert. Die vereinigten organischen Phasen werden 2 x mit Wasser und 2 x mit gesättigter NaCl-Lösung gewaschen, mit $MgSO_4$ getrocknet und eingedampft.

Die Chromatographie an Kieselgel (Hexan/Ether = 4:1) ergibt 1,24 g (8 mmol) Alkohol I (80 % Ausbeute), 90 % ee bestimmt nach Methode C.

Beispiel 32:

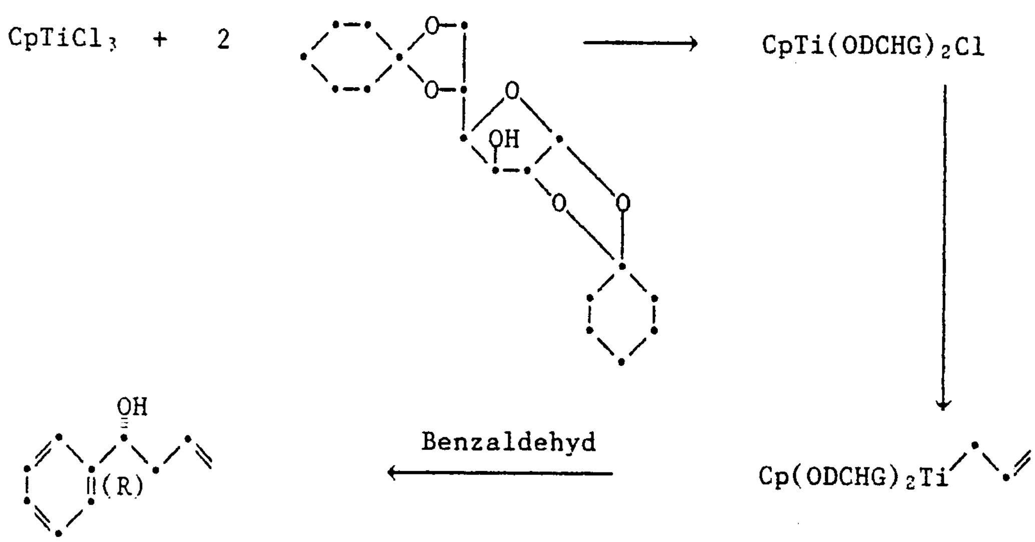


IV

2,15 g (9,8 mmol) Cyclopentadienyltitantrichlorid werden in 60 ml Diethylether gelöst und bei RT mit 6,33 g (19,6 mmol) Dicyclohexylidenglucose versetzt. 2,8 ml (20 mmol) Triethylamin, gelöst in 25 ml Diethylether, werden innert 30 Minuten zugetropft. Nach 2 Stunden wird das gebildete Triethylaminhydrochlorid unter

Argon abfiltriert.

Das Filtrat wird bei 0 °C mit 3,8 ml (9,8 mmol) Allylmagnesiumchlorid (2,6 m Lösung in Tetrahydrofuran) versetzt und 1 Stunde nachgerührt.

Bei -74 °C wird 0,89 ml (8,8 mmol) Benzaldehyd zugegeben und 2 Stunden nachgerührt. Die Lösung wird mit 30 ml 45%iger wässriger $NH_4F$-Lösung versetzt, filtriert und mit Ether extrahiert. Die organische Phase wird 2 x mit gesättigter NaCl-Lösung gewaschen, mit $MgSO_4$ getrocknet und eingedampft. Die Chromatographie an Kieselgel (Hexan/Ether = 5:1) ergibt 0,72 g (4,9 mmol) Alkohol IV (56 % Ausbeute); 75% ee bestimmt nach Methode A [$[\alpha]_D$ = 36,7 °C, (C6,22, Benzol)].

Beispiel 33:

Analog Beispiel 32 wird die Umsetzung mit 1,2:4,5-Di-O-Isopropylidene-D-fructopyranose (HDIFP) anstelle von Dicyclohexylidenglucose durchgeführt. Man erhält 0,7 g (4,7 mmol) Alkohol IV (75 % Ausbeute); 52 % ee bestimmt nach Methode A [$[\alpha]_D$ = 25,1 °C, (C6,22, Benzol)].

Beispiel 34:

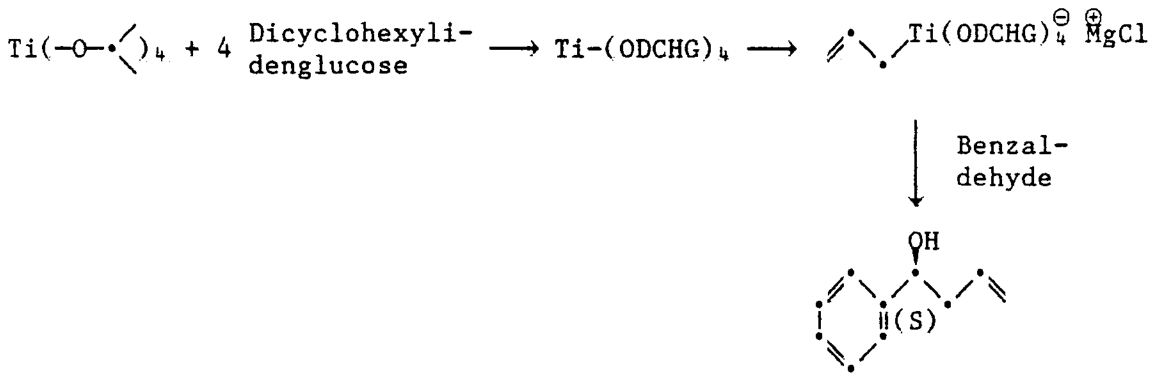


3,7 ml (12,5 mmol) Tetraisopropylorthotitanat und 17,0 g (50 mmol) Dicyclohexylidenglucose werden in 400 ml Cyclohexan gelöst. Dann werden unter Normaldruck 250 ml abdestilliert (Cyclohexan und 50 mmol Isopropanol). Unter HOchvakuum wird zur Trockene eingedampft und der Rückstand in 100 ml Diethylether gelöst. Die Lösung wird bei 0 °C mit 8 ml (10 mml) Allylmagnesiumchlorid (1,25 molar in Tetrahydrofuran) versetzt und 1 Stunde nachgerührt.

Bei -74 °C wird 0,81 ml (8 mmol) Benzaldehyd zugegeben und 2 Stunden nachgerührt. Die Lösung wird mit 30 ml 45%iger wässriger $NH_4F$-Lösung versetzt, filtriert und mit Ether extrahiert. Die organisiche Phase wird 2 x mit gesättigter NaCl-Lösung gewaschen, mit $MgSO_4$ getrocknetund eingedampft.

Die Chromatographie an Kieselgel (Hexan-Ether = 5:1) ergibt 0,64 g (4,32 mmol) (S)-1-Phenyl-3-buten-1-ol) (54 % Ausbeute); 77 % ee bestimmt nach Methode C.

Beispiel 35:

Analog Beispiel 34 wird die Umsetzung mit Diacetonglucose anstelle von Dicyclohexylidenglucose durchgeführt. Man erhält 0,75 g (5,07 mmol) (S)-1-Phenyl-3-buten-1-ol (63 % Ausbeute); 55 % ee bestimmt nach Methode C.

Beispiel 36:

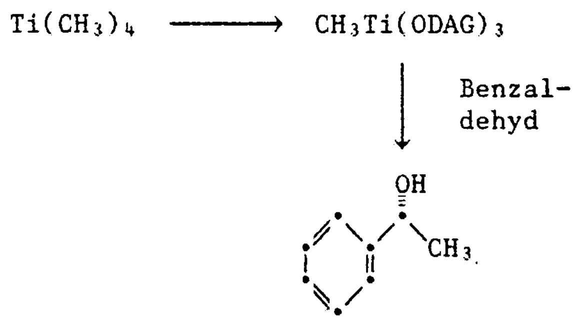


60 m Diethylether wreden bei -74 °C mit 1,10 ml (10 mmol) Titantetrachlorid versetzt (gelbe Suspension). 25 ml (40 mmol) Methyllithium (1,6 molar in Diethylether) werden innert 15 Minuten zugetropft; die grüne Lösung wird 30 Minuten bei -74 °C nachgerührt. Während 70 Minuten werden 7,81 g (30 mmol) Diacetonglucose in 150 ml Diethylether zugetropft. Nach weiteren 10 Minuten wird 0,90 mol (9 mmol) Benzaldehyd zugegeben und über Nacht auf RT aufgewärmt.

Die Lösung wird mit 30 ml 45%iger wässriger $NH_4F$-Lösung versetzt, filtriert und mit Ether extrahiert. Die organische Phase wird 2 x mit gesättigter NaCl-Lösung gewaschen, mit $MgSO_4$ getrocknet und eingedampft. Die Chromatographie an Kieselgel (Hexan/Ether = 1:5) ergibt 0,8 g (6,55 mmol) R-(+)-1-Phenylethanol (82 % Ausbeute); 62 % ee bestimmt nach Methode C.

Beispiel 37: (3R)-Hydroxycaprinsäure

70 mmol Lithiumdicyclohexylamid in 150 ml ether [14,50 g (80 mmol) Dicyclohexylamin; 43,7 ml (70 mmol) 1,6 M Lithium-Butyl in Hexan] werden unter Argon bei -73 °C vorgelegt.

6,96 g (60,0 mmol) Essigsäure-tert-butylester werden in 842 ml (80 mmol) einer 0,095 M ClTicp-$(ODAG)_2$-Toluol-Lösung gelöst und innert 4 Stunden bei -72 bis -70 °C zur Lithiumdicyclohexylamid-Lösung zugetropft.

Die Reaktionslösung wurde 1 Stunde bei -73 °C gerührt und langsam (30 Minuten) auf -35 °C erwärmt. Nach 30-minütigem Rühren bei -35 °C wird die Lösung auf -74 °C abgekühlt und 7,69 g (60,0 mmol) Octanal (Caprylaldehyd) in 35 ml Ether innert 45 Minuten zugetropft.

Die Reaktionslösung wird 45 Minuten bei -74 °C gerührt und mit 150 ml 45%iger wässriger $NH_4F$-Lösung versetzt. Die Lösung wird filtriert und mit Ether extrahiert. Die organische Phase wird 2 x mit gesättigter NaCl-Lösung gewaschen, mit $MgSO_4$ getrocknet, filtriert und eingedampft.

Das Rohprodukt (58,46 g gelbliche, ölige Kristalle) wird in 1 l 0,1 n HCl aufgeschlämmt und 1,5 Stunden bei RT gerührt.

Die Wasserphase wird 4 x mit Ether (150 ml) extrahiert. Die vereinigten organischen Phasen werden mit je 2 x 50 ml Wasser und je 2 x 50 ml gesättigter NaCl-Lösung gewaschen, mit $MgSO_4$ getrocknet, filtriert und eingedampft.

Das Rohprodukt (14,32 g gelbes Oel) wird destilliert (93 °C/0,02 mbar) und ergibt 12,56 g (51,40 mmol; 85 %) Ester (3R)-Hydroxycaprinsäure-t-butylester als gelbliches Oel.

5,11 g (20,91 mmol) des Esters werden bei 0 °C mit 30 ml Trifluoressigsäure versetzt und 1,5 Stunden bei RT am Vakuum (~ 50 mbar) gerührt. Die Reaktionslösung wird mit 50 ml Toluol verdünnt und eingedampft. Das Rohprodukt wird noch 2 x mit je 30 ml Toluol eingedampft. Man erhält 4.08 g braune Kristalle, 95 % ee (Methode C). Eine Kristallisation aus 25 ml Cyclohexan ergibt 1,58 g (8,39 mmol: 40 %) farblose Kristalle und aus der Mutterlauge isolierte man 2,15 g (11,42 mmol; 54 %) gelbliche Kristalle.

Beispiel 38: (2R,3S)-2,4-Dimethyl-3-hydroxy-pentansäure(2′,6′-dimethylphenylester

Zu einer Lösung von 0,4 ml (2,4 mmol) Cyclohexylisopropylamin (frisch über $CaH_2$ destilliert) in 10 ml Ether werden bei -20 °C 1,42 ml (2,2 mmol) einer Lösung von Lithium-n-butyl in Hexan zugetropft. Nach 15 Minuten werden bei -78 °C 357 mg (2 mmol) Propionsäure-2,6-dimethylphenylester, gelöst in 30 ml einer 0,085 M Lösung von $ClTiCp(ODAG)_2$ in Ether während 1,5 Stunden zugetropft. Nach 2 Stunden bei -78 °C und 30 Minuten bei -30 °C werden bei -78 °C 0,18 ml (2 mmol) 2-Methylpropanal zugegeben. Nach 2

Stunden bei -78 °C werden 40 ml 45%ige wässrige $NH_4F$-Lösung zugetropft, das Kältebad entfernt und sodann bei RT mit 30 ml 2N HCl versetzt. Die klare wässrige Phase wird im Scheidetrichter abgetrennt und einmal mit Ether extrahiert. Die vereinigten organischen Phase wurden mit gesättigter NaCl-Lösung gewaschen mit $MgSO_4$; getrocknet und eingedampft.

Der erhaltene Rückstand wird mit 70 ml 0,1 N HCl versetzt, während 1,5 Stunden stark gerührt und anschliessend zweimal mit Ether extrahiert. Die organischen Phasen werden mit gesättigter NaCl-Lösung gewaschen, mit $MgSO_4$ getrocknet und eingedampft. Flash-Chromatographie des Rohprodukts an $SiO_2$ mit Hexan/Essigester = 5:1 ergibt 230 mg (46 %) (2R,3S)-2,4-Dimethyl-3-hydroxy-pentansäure-2,6-dimethylphenylester, Mp: 58,5 - 59,5 °C.
$[\alpha]_D^{RT}$ : + 15,7° (c = 1, $CHCl_3$)
ee ≧ 95 % (Methode C). Die durch basische Hydrolyse hergestellte Carbonsäure zeigt ein $[\alpha]_D^{RT}$ von + 10,6 °C (c = 0,16, $CHCl_3$).

Beispiel 39: (+)-2-Fluor-2-Phenyl-3-hydroxypentansäure-2′,6′-dimethylphenolester

Mit einer 1,6 molaren Butyllithium-Hexan-Lösung und 0,255 g Diisopropylamin in 10 ml THF werden unter Schutzgas bei -30 °C bis Raumtemperatur 2,5 mmol Lithiumdiisopropylamid hergestellt. Bei -78 °C werden 2,0 mmol racemischer 2-Fluor-2-phenylessigsäure-2′,6′-dimethylphenylester (hergestellt durch DAST-Fluorierung von racemischer Mandelsäureethylester und Umesterung mit 2,6-Dimethylphenol) in 1 ml Tetrahydrofuran langsam zugetropft und anschliessend bei gleichen Bedingungen mit 23 ml einer 0,1 molaren Etherlösung von $CpTi(ODAG)_2Cl$ versetzt. Nach 2 Stunden Rühren werden bei -78 °C 7 mmol frisch destilliertes Propionaldehyd in wenig Tetrahydrofuran zugetropft und die Reaktionslösung allmähliche auf RT erwärmt. Nach hydrolytischer Aufarbeitung (4N HCl) und Etherextraktion wird der Rückstand mittels Flashchromatographie (Hexan/Essigester = 9:1) an Kieselgel gereinigt. Nach Eindampfen der vereinigten Produktfraktionen werden 0,250 g (40 %) eines halbkristallinen 7:1 -Diastereoisomerengemisches von 2-Fluor-2-phenyl-3-hydroxypentansäure-2′,6′-dimethylphenylester isoliert. Die beiden Diastereoisomeren lassen sich leicht anhand der Methylgruppensignale im $^1$H-NMR-Spektrum unterscheiden.

Das Hauptdiastereoisomer zeigt folgende Resonanzlagen im 300 MHz $^1$H-NMR-Spektrum ($CDCl_3$): 7,66 ppm (m, 2 arom. H); 7,45 ppm (m, 3 arom. H); 7,00 ppm (s, br., 3 arom. H); 4,41 ppm (m, 1H, C<u>H</u>-OH); 1,88 (s, br., 6H, arom. $CH_3$); 1,76 ppm (m, 2H, C<u>H</u>$_2$); 1,13 ppm (t, j = 7 Hz, 3H, C<u>H</u>$_3$).

Das Nebendiastereoisomer unterscheidet sich signifikant in folgenden Resonanzsignalen: 4,50 pp (m, 1H, C<u>H</u>-OH); 1,92 ppm (s, br., 6H, arom. $CH_3$); 1,45 ppm (m, 2H, C<u>H</u>$_2$); 1,02 ppm (t, j = 7Hz, 3H, C<u>H</u>$_3$).

Die Enantiomerenüberschüsse bei beiden Diastereoisomeren konnte mittels $^1$H-NMR-Shift-Experimenten (Methode D) bestimmt werden. Analysiert wurden die ee-Werte anhand der $\delta_\Delta$-Werte der aliphatischen $CH_3$-Gruppen bei 1,13 und 1,02 ppm:
ee-Wert der Hauptdiastereoisomeren: ≧ 90 %
ee-Wert der Nebendiastereoisomeren: ≧ 50 %
$[\alpha]_D^{25}$ des Hauptdiastereomeren = + 12,03 (c = 1,005, EtOH)

Beispiel 40: α-Amino-β-hydroxy-capronsäureethylester

Unter Luftausschluss wird eine Lösung von 0,75 ml frisch über $CaH_2$ destilliertem Cyclohexylisopropylamin (4,49 mmol) in 20 ml Tetrahydrofuran bei -40 bis -35 °C mit 2,5 ml Lithium-n-butyl (1,6 M in Hexan, 4,08 mmol) versetzt, 20 Minuten bei dieser Temperatur gehalten und dann auf -78 °C abgekühlt. Dazu tropft man eine Lösung von 1,0 g des geschützten Glycinethylesters* in 20 ml Tetrahydrofuran, rührt 1 Stunde nach, setzt den Zucker-Titan-Komplex zu (54 ml $ClCpTi(ODAG)_2$, 0,09 M in Ether; 4,9 mmol), rührt wiederum 1 Stunde nach und versetzt die braune Reaktionslösung mit 0,405 ml Butyraldehyd (4,49 mmol) in 15 ml Tetrahydrofuran. Es wird während 22 Stunden bei -78 °C gerührt und dann durch Zugabe von 50 ml Pufferlösung (0,41N $Na_2HPO_4$; 0,28N $KH_2PO_4$) bei -78 °C hydrolysiert und auf RT aufgewärmt. Eine Verteilung des Reaktionsgemisches zwischen 3 Portionen Ether, 2 Portionen Wasser und schliesslich gesättigter NaCl-Lösung ergibt ein teilweise kristallines Gemisch von Reaktionsprodukt und Diacetonglucose. Durch eine weitere Verteilung dieses Gemisches zwischen 3 Portionen Hexan und 4 Portionen $CH_3CN/H_2O$ (4:1) wird die Diacetonglucose quantitativ vom Reaktionsprodukt (in Hexan-Phase) abgetrennt. Man erhält 970 mg Oel.

Das Rohprodukt wird gelöst in 25 ml Tetrahydrofuran, 5 ml $H_2O$ und 1 ml Essigsäure und während 3 Stunden bei RT gerührt. Das Hydrolysegemisch wird eingedampft und chromatographiert (Kieselgel; $CH_2Cl_2/EtOH/Et_3N$ 93:5:2): 164,4 mg (23 %) reines threo-Produkt, gefolgt von 124,3 mg (17 %) eines Gemisches von Threo- und Erythroprodukt.

Analytische Daten des Threoproduktes: Schmelzpunkt 59 - 61 °C; ee 95 % (Methode D und I), $[\alpha]_D^{25}$ = -16,3 ° (c = 0,959, Ethanol).
$^1$H-NMR: ($CDCl_3$) 0,95 (t, J = 7, 3H, $H_3C$), 1,30 (t, J = 7, 3H, $H_3C$), 1,3 - 1,6 (m, 4H, $2H_2C$), 2,26 (sb, 3H, HO, $H_2N$), 3,36 (d, J = 5, 1H, $HC_\alpha$), 3,74 - 3,82 (m, 1H, $HC_\beta$), 4,22 (q, J = 7, 2H, $H_2C$).

Analytische Daten des Erythroproduktes:
$^1$H-NMR: ($CDCl_3$) 0,93 (t, J = 7, 3H, $H_3C$), 1,30 (t, J = 7, 3H, $H_3C$), 1,3 - 1,6 (m, 4H, $2H_2C$), 2,90 (sb, 3H, HO, $H_2N$), 3,64 (d, J = 4, 1H, $HC_\alpha$), 3,89 - 3,91 (m, 1H, $HC_\beta$), 4,15 - 4,30 (m, 2H, $H_2C$).

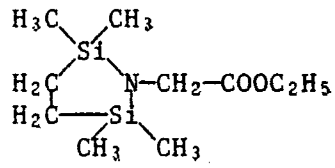

Die threo/erythro-Zuordnung erfolgt auf der Stufe der freien Säuren aufgrund der chemischen Verschiebungen des $H_\alpha$ im $^1$H-NMR-Spektrum [Lit, Y. Ariyoshiand, N. Sato, Bull. Chem. Soc. Japan 44, 3435 (1971)].

Beispiel 41: Herstellung von (S)-(-)-Ipsenol

a) 88 g (0,4 mol) Cyclopentadienyltitantrichlorid werden unter Argon in 3 l absolutem Toluol vorgelegt und anschliessend mit 208,2 g (0,8 mol) Diacetonglucose (frisch sublimiert) versetzt. Zu diesem Gemisch werden bei Raumtempertur innerhalb 1 Stunde 83,0 g (0,82 Mol) Triethylamin, gelöst in 500 ml absolutem Toluol, zugetropft. Nach 5 Stunden kräftigem Rühren wird das ausgefallene Aminhydrochlorid unter Argon über Celite abfiltriert und der Niederschlag mit wenig absolutem Toluol gewaschen. Man erhielt eine Lösung von 4186 ml des Zuckerkomplexes gemäss Beispiel 1a, die direkt für den folgenden Reaktionsschnitt eingesetzt wird.

b) Herstellung von

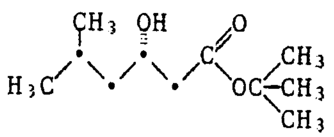

69 g (0,38 mol) Dicyclohexylamin werden in 1 l Diethylether gelöst, auf -30 °C gekühlt und bei dieser Temperatur mit 225 ml einer 1,6 molaren n-Butyl-Lithium-Lösung in Hexan innerhalb 40 Minuten versetzt. Nach 30 Minuten Rühren bei -25 °C wird das Gemisch auf -78 °C gekühlt. Dazu werden innerhalb 3 Stunden 3977 ml der Toluol-Lösung a), vermischt mit 40,7 g (0,35 mol) Essigsäure-t-butylester, bei -70 - 78 °C zugetropft. Anschliessend wird das Reaktionsgemisch innerhalb 1 Stunde auf -30 °C erwärmt, 30 Minuten bei dieser Temperatur gehalten und dann erneut auf -78 °C abgekühlt. Innerhalb 45 Minuten werden bei dieser Temperatur 30,3 g (0,35 mol) Isovaleraldehyd, gelöst in 100 ml Diethylether, zugetropft; dann wird die Reaktionslösung weitere 90 Minuten bei -78 °C gerührt und anschliessend mit 1 l einer 45%igen wässrigen Ammoniumfluorid-Lösung versetzt. Der entstandene Niederschlag wird abfiltriert und mit 2 x 100 ml Toluol gewaschen. Die wässrige Phase wird von der organischen Phase abgetrennt und 2 x mit 250 ml Ether extrahiert. Anschliessend werden die vereinigten organischen Phasen mit 3 x 100 ml Wasser gewaschen mit Magnesiumsulfat getrocknet und am Rotationsverdampfer eingedampft. Der Rückstand (307 g) wird mit 4,5 l 0,1 N Salzsäure versetzt und während 2 Stunden kräftig gerührt. Das erhaltene Gemisch wird im Scheidetrichter 3 x mit 600 ml Ether extrahiert. Die vereinigten Etherphasen werden über Celite von einem geringen Niederschlag abgetrennt und anschliessen mit Magnesiumsulfat getrocknet. Das Lösungsmittel wird im Vakuum abgezogen und der Rükckstand destilliert; man erhält 37,1 g des gewünschten 3-Hydroxy-t-Butylesters als farblose Flüssigkeit mit einem Siedepunkt von 53 - 54 °C (0,39 mbar) (53 % der Theorie); $[\alpha]_D^{25}$ + 14,39 ° (c = 1,529 in $CHCl_3$), 96 % ee (Methode A und B).

c) Herstellung von

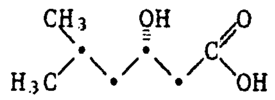

37,1 g (0,183 mol) β-Hydroxy-t-Butylester gemäss b) werden bei 0 °C mit 200 ml Trifluoressigsäure versetzt. Das Gemisch wird bei reduziertem Druck (65 - 130 mbar) während 1 Stunde bei Raumtemperatur gerührt. Anschliessend wird die Trifluoressigsäure im Vakuum entfernt und die erhaltene rohe β-Hydroxysäure gereinigt. Der Rückstand wird in 150 ml 2N Natronlauge gelöst und mit 150 ml Ether extrahiert. Die basische Phase wird mit 10N Salzsäure sorgfältig auf pH 3 angesäuert und 4 x mit 400 ml Methylenchlorid extrahiert. Die organische Phase wird mit Magnesiumsulfat getrocknet und das Lösungsmittel im Vakuum entfernt. Man erhält 22.8 g (0,156 mol) der gewünschten Hydroxysäure (85 % der Theorie). Durch Umkristallisation aus 1,25 l Cyclohexan erhält man 19,35 g (0,132 mol) enantiomerenreine (+)-R-2-Hydroxysäure (nach Methode C) mit einem Schmelzpunkt von 82 - 83 °C (38 % der Theorie bezogen auf eingesetzte Menge Isovaleraldehyd, $[\alpha]_D^{25}$ + 15,15 ° (c = 1,023 in $CHCl_3$).

d) Herstellung von

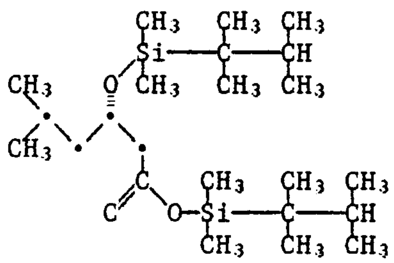

9,4 g (0,064 mol) β-Hydroxysäure c) werden in 35 ml Dimethylformamid gelöst und mit 12,71 g (0,187 mol) Imidazol und 26,47 g (0,148 mol)Dimethyl(2,3-dimethyl-2-butyl)chlorsilan (Thexyldimethylchlorsilan) versetzt. Nach 24 Stunden Rühren wird das Reaktionsgemisch auf 200 ml Wasser gegossen und mit 200 ml Hexan extrahiert. Die wässrige Phase wird zusätzlich mit 2 x 50 ml Hexan extrahiert. Die vereinigten organischen Phasen werden mit Magnesiumsulfat getrocknet und anschliessen im Vakuum eingedampft. Der erhaltene Rückstand wird abfiltriert. Man erhielt 26,97 g (0,063 mol) Produkt als farblose Flüssigkeit mit einem Siedepunkt von 119-120 °C (0,0325 mbar) (98 % der Theorie).

$[\alpha]_D^{25}$ + 9,19 ° (c = 1,07 in Toluol.

e) Herstellung von

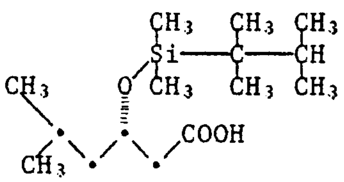

21,5 g (0,05 mol) bissilylierte Hydroxysäure d) werden in 200 ml Tetrahydrofuran gelöst und mit 100 ml Wasser und 100 ml 1N $NaHCO_3$-Lösung versetzt. Das Zweiphasensystem wird während 24 Stunden kräftig gerührt, dann mit 500 ml Wasser versetzt und mit 75 ml 2N Salzsäure vorsichtig angesäuert. Zur besseren Phasentrennung werden 300 ml Ether zugegeben. Die organische Phase wird im Scheidetrichter abgetrennt, mit Magnesiumsulfat getrocknet und im Vakuum eingedampft. Man erhält ein Gemisch von Silanol und der gewünschten Thexyldimethylsilyl geschützten β-Hydroxysäure. Durch Erwärmen des Gemisches auf 50 °C bei reduziertem Druck (0,1 mm Hg) wird Thexyldimethylsilanol praktisch vollständig abdestilliert. Durch Chromatographie an Kieselgel mit Hexan/Essigester 4:1 wird 2-Thexyldimethylsilyloxy-4-Methyl-capronsäure rein isoliert: 12,96 g (90 % der Theorie) farblose Flüssigkeit.

$^1$H-NMR: (60 MHz, $CDCl_3$); 0,18 (s, 6H), 0,82 (d, 6H), 0,88 (d, 12H), 1,2 - 1,5 (m, 3H), 1,6 (m, 1H), 2,4 (dd, 2H), 4,1 (m, 1H). $[\alpha]_D^{25}$ = -2,62 ° (c = 0,994 in $CHCl_3$).

f) Herstellung von

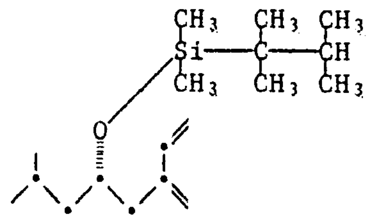

16,82 g (0,0584 mol) Säure e) werden in 50 ml Methylenchlorid gelöst und unter Rühren bei 0 °C mit 9,08 ml (0,0642 mol) 1 Chlor-1-Dimethylamino-2-Methyl-prop-1-en versetzt. Das Reaktionsgemisch wird langsam auf Raumtempertur erwärmt. Nach weiteren 30 Minuten Rühren wird das Lösungsmittel im Vakuum entfernt, der Rückstand in 100 ml Ether aufgenommen und bei -30 °C mit 11,68 g (0,0613 mol) Cu(I)J versetzt. Die erhaltene weisse Suspension wird weiter auf -78 °C gekühlt und mit 64,4 ml (0,082 mol) einer 1,25 m etherischen Lösung von Trimethylsilylmethylmagnesiumchlorid umgesetzt. Das Reaktionsgemisch wird langsam auf 0 °C erwärmt und während 1 Stunde bei dieser Temperatur weitergerührt. Dann werden sorgfältig 11,56 ml einer 5,05 m Ammoniumchlorid-Lösung zugetropft und die ganze Mischung über Hyflo filtriert. Das klare Filtrat wird mit Magnesiumsulfat getrocknet und mit 50 ml Benzol versetzt. Dann wird das Lösungmittel im Vakuum entfernt und der Rückstand ohne weitere Reinigung für die folgende Reaktion eingesetzt.

10,47 g des Rückstandes werden in 50 ml Tetrahydrofuran gelöst und bei 0 °C tropfenweise mit 64,7 ml einer 1 m Vinyl-magnesiumbromid Lösung versetzt. Anschliessend wird das Reaktionsgemisch bei Raumtemperatur während 18 Stunden gerührt. Dann wird durch Zugabe eines Gemisches von 150 ml gesättigter Ammoniumchlorid-Lösung und 25 ml 2N Salzsäure das überschüssige Grignardreagens zerstört. Extraktion mit 3 x 50 ml Ether ergibt nach Trocknung der organischen Phasen mit Magnesiumsulfat und Abziehen des Lösungsmittel im Vakuum 9,8 g einer leicht gelben Flüssigkeit, die direkt im folgenden Reaktionsschritt eingesetzt wird.

Der Rückstand (9,8 g) wird mit 50 ml mit Natriumacetat gesättigter Essigsäure versetzt und bei 50 °C während 5 Stunden gerührt. Dann wird das Reaktionsgemisch auf 400 ml Eis/Wasser gegossen und mit 3 x 100 ml Hexan extrahiert. Die vereinigten organischen Phasen werden 2 x mit 30 ml gesättigter $NaHCO_3$-Lösung gewaschen und mit Magnesiumsulfat getrocknet. Nach Abziehen des Lösungsmittels im Vakuum wird der Rückstand (18,7 g) an 150 g Kieselgel chromatographisch gereinigt (Eluierungsmittel Hexan). Man isoliert 7,41 g eines Produkts als farblose Flüssigkeit (43 % der Theorie bezogen auf die eingesetzte Menge Thexyldimethylsilyloxy-4-Methylcapronsäure). Kp 88 - 90 °C (0,13 mbar)

$[\alpha]_D^{25}$ - 11,5 ° (c = 1,01 in Ethanol).

g) Herstellung von (-)-(S)-Ipsenol

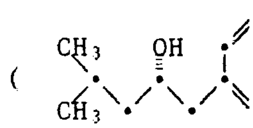

7,41 g (0,025 mol) Thexyldimethylsilyl geschützten (-)-S-Ipsenol gemäss f) werden in 100 ml Tetrahydrofuran mit 23,7 g (0,075 mol) Tetrabutylammoniumfluorid-Trihydrat versetzt und während 40 Stunden bei Raumtemperatur gerührt. Das Reaktionsgemisch wird dann mit 200 ml Wasser versetzt und mit 3 x 100 ml Ether extrahiert. Die vereinigten Etherphasen werden mit 20 ml gesättigter Natriumchlorid-Lösung gewaschen und anschliessend mit Magnesiumsulfat getrocknet. Dann wird das Lösungsmittel im Vakuum entfernt und der Rückstand destilliert. Man erhält 4,1 g einer farblossen Flüssigkeit mit einem Siedepunkt 76 - 85 °C (6,5 mbar), die mit ca. 15 - 20 % Thexyldimethylsilanol verunreinigt ist. Nach Chromatographie an 200 g Alox mit Methylenchlorid als Lösungsmittel isoliert man 3,48 g reines (-)-S-Ipsenol (90 % der Theorie) mit einem Siedepunkt von 78 - 79 °C (6,5 mbar).

$[\alpha]_D^{25}$ - 17,62 ° (c = 1,028 in Ethanol).

**Patentansprüche**

**Patentansprüche für folgende Vertragsstaaten : BE, CH, DE, FR, GB, IT, LI, LU, NL, SE**

1. Verbindungen der Formeln I und Ia

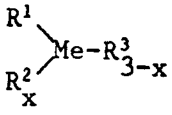 (I) und 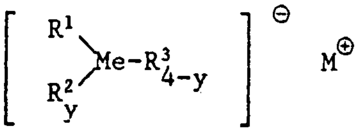 (Ia),

worin

Me für vierwertiges Titan, Zirkonium oder Hafnium steht,

$R^1$ lineares oder verzweigtes Alkyl, Alkenyl, Alkinyl, gegebenenfalls durch $C_1$-$C_4$-Alkyl substituiertes Cycloalkyl oder Cycloalkenyl, Aryl, Alkaryl, Aralkyl, Alkaralkyl, Aralkenyl, Alkaralkenyl, Aralkinyl oder Alkaralkinyl bedeuten, die gegebenenfalls ein- oder mehrfach durch $(C_6H_5)_2P$-, $(R^5O)_2P(O)$-, $R^5_3$ Si-, $R^5_3$ SiO-, $R^5SO_2$-, -S-$C_2$-$C_4$-Alkylen-S-, -O-$C_2$-$C_4$-Alkylen-O-, wobei $R^5$ für Phenyl, Benzyl oder $C_1$-$C_8$-Alkyl steht, Cyano, F, Nitro, $C_1$-$C_{12}$-Alkylthio, $C_1$-$C_{12}$-Alkoxy, Sekundäramino oder -$COR^4$, worin $R^4$ der Rest eines einwertigen Alkohols ist, substituiert sind; oder einen Rest eines Enols, Enamins oder Enhydrazins bedeutet;

$R^2$ gegebenenfalls durch Alkyl, Alkenyl, Alkoxy, Cycloalkyl, Aryl, Aralkyl, Trialkoxysilyl, Trialkylsilyl oder Halogen substituiertes Cyclopentadienyl, Alkoxy, Cycloalkoxy, Cycloalkylalkoxy, Aryloxy, Aralkyloxy, Alkylthio, Arylthio, Aralkylthio; Halogen, Pseudohalogen, Acyloxy, Acylamino oder Trialkylsilyloxy darstellt,

$R^3$ der um ein Hydroxyl-, Thiol- oder Aminwasserstoffatom verminderte Rest eines geschützten, monohydroxyfunktionellen, monothiolfunktionellen oder monoaminfunktionellen, optisch aktiven Zuckers, Thiozuckers oder Aminozuckers, oder deren Derivaten aus der Gruppe der Zuckeralkohole; Ester einer Zuckersäure, Aldozuckersäure oder Ketozuckersäure; Aminozucker,

Zuckermercaptale oder Desoxyzucker ist,

x für 0, 1 oder 2 und y für 0, 1, 2 oder 3 und

$M^{\oplus}$ für $Li^{\oplus}$, $Na^{\oplus}$, $K^{\oplus}$, $MgY^{\oplus}$, $ZnY^{\oplus}$, $CdY^{\oplus}$, $HgY^{\oplus}$, $CuY^{\oplus}$ oder quaternäres Ammonium stehen, worin Y Halogen bedeutet.

2. Verbindungen gemäss Anspruch 1, worin $R^1$ gegebenenfalls ein- oder mehrfach durch Sekundäramino, Cyano, Nitro, $C_1$-$C_6$-Alkylthio, $C_1$-$C_6$-Alkoxy oder -$COR^4$, worin $R^4$ $C_1$-$C_{12}$-Alkoxy ist, substituiertes lineares oder verzweigtes $C_1$-$C_{18}$-Alkyl, C-$_2$-$C_{12}$-Alkenyl, $C_2$-$C_{12}$-Alkinyl, Cycloalkyl mit 3-8 Ring-C-Atomen, Cycloalkenyl mit 3 bis 8 Ring-C-Atomen, $C_6$-$C_{12}$-Aryl, $C_7$-$C_{16}$-Alkaryl oder Aralkyl, $C_8$-$C_{16}$-Alkaralkyl, $C_8$-$C_{16}$-Aralkenyl, $C_9$-$C_{16}$-Alkaralkenyl, $C_8$-$C_{16}$-Aralkinyl, $C_9$-$C_{16}$-Alkaralkinyl; oder ein über das Enolsauerstoffatom oder über das Enaminstickstoffatom gebundener Rest eine Enols, Enamins oder Enhydrazins.

3. Verbindungen gemäss Anspruch 2, worin $R^1$ gegebenenfalls ein- oder mehrfach durch Sekundäaramino, Cyano, Nitro, $C_1$-$C_6$-Alkoxy, $C_1$-$C_6$-Alkylthio oder -$COR^4$, worin $R^4$ $C_1$-$C_{12}$-Alkoxy ist, substituiertes lineares oder verzweigtes $C_1$-$C_6$-Alkyl, $C_2$-$C_6$-Alkenyl, $C_2$-$C_6$-Alkinyl, Cycloalkyl mit 3-6 Ring-C-Atomen, Cycloalkenyl mit 3 bis 6 Ring-C-Atomen, Phenyl, ($C_1$-$C_{10}$-Alkyl)phenyl, Phenyl($C_1$-$C_2$-alkyl), ($C_1$-$C_8$-Alkyl)phenyl($C_1$-$C_2$-alkyl), Phenylvinyl, Phenylethinyl oder Phenylpropargyl, ($C_1$-$C_8$-Alkyl)phenylvinyl, ($C_1$-$C_8$-Alkyl)phenylethinyl oder ($C_1$-$C_7$-Alkyl)phenylpropargyl; oder ein über ein Enolsauerstoffatom oder Enaminostickstoffatom gebundener Rest eines Enols, Enamins oder Enhydrazins mit bis zu 20 C-Atomen.

4. Verbindungen gemäss Anspruch 2, worin $R^1$ gegebenenfalls ein- oder Sekundäramino, Cyano, Nitro, $C_1$-$C_6$-Alkoxy, $C_1$-$C_6$-Alkylthio oder -$COR^4$, worin $R^4$ $C_1$-$C_{12}$-Alkoxy ist, substituiertes lineares oder verzweigtes $C_1$-$C_4$-Alkyl, Vinyl, Allyl, Ethinyl, Propargyl, Cyclopentyl, Cyclohexyl, Cyclopentenyl und Cyclohexenyl, Phenyl, Methylphenyl, Benzyl, 1-Phenyleth-2-yl, Methylbenzyl, Phenylvinyl, Methylphenylvinyl, Phenylethinyl, Phenylpropargyl, Methylphenylethinyl, Dimethylphenylethinyl oder -propargyl; oder ein über das Enolsauerstoffatom oder über das Enaminostickstoffatom gebundener Rest eines Enols, Enamins oder Enhydrazins mit 2-16 C-Atomen.

**5.** Verbindungen gemäss Anspruch 1, worin $R^2$ gegebenenfalls durch $C_1$-$C_6$-Alkyl, $C_2$-$C_6$-Alkenyl, $C_1$-$C_6$-Alkoxy, Cycloalkyl mit 5 oder 6 Ring-C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, oder Br substituiertes Cyclopentadienyl, $C_1$-$C_{18}$-Alkoxy, $C_6$-$C_{12}$-Aryloxy, $C_7$-$C_{16}$-Aralkyloxy, $C_1$-$C_{18}$-Alkylthio, $C_6$-$C_{12}$-Arylthio, $C_7$-$C_{16}$-Aralkylthio; Halogen, Pseudohalogen, $C_1$-$C_{18}$-Acyloxy, $C_1$-$C_{18}$-Acylamino oder Trialkylsilyloxy mit 1 bis 6 C-Atomen in den Alkylgruppen darstellt.

**6.** Verbindungen gemäss Anspruch 5, worin $R^2$ gegebenenfalls wie in Anspruch 5 angegeben substituiertes Cyclopentadienyl, $C_1$-$C_6$-Alkoxy, Phenoxy, Benzyloxy, $C_1$-$C_6$-Alkylthio, Phenylthio, Benzylthio; Cl, Br, J, CN, CNS, CNO, $C_1$-$C_{12}$-Acyloxy, $C_1$-$C_{12}$-Acylamino oder Trialkylsilyloxy mit 1 bis 4 C-Atomen in den Alkylgruppen darstellt.

**7.** Verbindungen gemäss Anspruch 1, worin $R^3$ der Rest eines geschützten monohydroxy-, monothiol- oder monoaminofunktionellen $C_3$-$C_7$-Monosaccharids oder entsprechender Di- oder Trisaccharide oder deren Derivaten aus der Gruppe der Zuckeralkohole; Ester einer Zuckersäure, Aldozuckersäure oder Ketozuckersäure; Aminozucker, Desoxyzucker oder Zuckermercaptale ist.

**8.** Verbindungen gemäss Anspruch 7, worin $R^3$ den Rest eines $C_5$- oder $C_6$-Monosaccharids oder dessen Derivate bedeutet.

**9.** Verbindungen gemäss Anspruch 7, worin $R^3$ der Rest einer geschützten Furanose oder Pyranose ist.

**10.** Verbindungen gemäss Anspruch 1, worin die Hydroxylgruppen der Zucker und deren Derivate durch $C_1$-$C_8$-Acyl, $C_1$-$C_8$-Alkyl, Benzyl, Diphenylmethyl, Trityl, $C_1$-$C_8$-Alkyliden, Triphenylsilyl oder Trialkylsilyl mit 1 bis 8 C-Atomen in den Alkylgruppen, $(C_6H_5)_2Si=$, $(C_1$-$C_8$-$Alkyl)_2Si$, $(C_6H_5)_2Sn=$ oder $(C_1$-$C_8$-$Alkyl)_2Sn=$ geschützt sind.

**11.** Verbindungen gemäss Anspruch 1, worin $R^3$ der um das Hydroxylwasserstoffatom verminderte Rest von 1,2:5,6-Di-O-isopropyliden-$\alpha$-glucofuranoseist.

**12.** Verbindungen gemäss Anspruch 1, worin x für 1 und y für 1 stehen.

**13.** Verbindungen gemäss Anspruch 1, worin Y Cl, Br oder J bedeuten.

**14.** Verbindungen gemäss Anspruch 1, worin $M^{\oplus}$ $Li^{\oplus}$, $MgCl^{\oplus}$, $MgBr^{\oplus}$, $ZnCl^{\oplus}$, $ZnBr^{\oplus}$, $CdCl^{\oplus}$, $CdBr^{\oplus}$ oder Tetraalkylammonium mit 1 bis 6 C-Atomen in den Alkylgruppen bedeutet.

**15.** Verbindungen gemäss Anspruch 1, worin Me für Ti steht, $R^1$ Allyl oder 1-(t-Butyloxy)-vinyl-1-oxyl bedeuten, $R^2$ Cyclopentadienyl ist und $R^3$ der um das Hydroxylwasserstoffatom verminderte Rest von 1,2:5,6-Di-O-isopropyliden-$\alpha$-glucofuranose ist.

**16.** Verfahren zur Herstellung von Verbindungen der Formel I und Ia gemäss Anspruch 1, dadurch gekennzeichnet, dass man eine Verbindung der Formel II

$$R^2_xR^3_{3-x}MeX \qquad (II)$$

oder eine Verbindung der Formel (IIa)

$$R^2_yR^3_{4-y}Me \qquad (IIa)$$

in Gegenwart eines inerten Lösungsmittels und eines inerten Schutzgases mit einer Verbindung der Formel III

$R^{1\ominus}M^{\oplus}$ (III)

umsetzt, wobei Me, $R^1$, $R^2$, $R^3$, $M^⊕$, x und y die in Anspruch 1 angegebene Bedeutung haben und X für ein Anion steht.

**17.** Verbindungen der Formel II

$$R^2_x R^3_{3-x} MeX \quad (II)$$

worin Me, $R^2$, $R^3$, und x die in Anspruch 1 angegebene Bedeutung haben.

**18.** Verwendung von Verbindungen der Formeln I oder Ia gemäss Anspruch 1 als enantioselektive Reaktanden für Verbindungen mit Carbonyl- und/oder N-substituierten Imingruppen.

**19.** Verfahren zur Herstellung von sekundären und tertiären Alkoholen und sekundären Aminen, dadurch gekennzeichnet, dass man Aldehyde, Ketone, N-substituierte Aldimine oder Ketimine mit 1 Mol einer Verbindung der Formel I oder Ia gemäss Anspruch 1 pro Mol Aldehyd-, Keto- oder Imingruppe umsetzt.

**20.** Verfahren gemäss Anspruch 19, dadurch gekennzeichnet, dass es bei Temperaturen von -80 bis 30 °C durchgeführt wird.

**Patentansprüche für folgende Vertragsstaaten: AT, GR, ES**

**1.** Verfahren zur Herstellung von Verbindungen der Formeln I und Ia

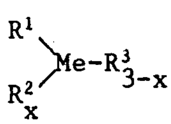 (I) und 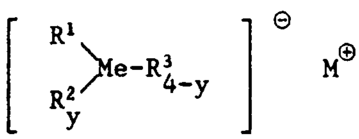 (Ia),

worin
Me für vierwertiges Titan, Zirkonium oder Hafnium steht,
$R^1$ lineares oder verzweigtes Alkyl, Alkenyl, Alkinyl, gegebenenfalls durch $C_1$-$C_4$-Alkyl substituiertes Cycloalkyl oder Cycloalkenyl, Aryl, Alkaryl, Aralkyl, Alkaralkyl, Aralkenyl, Alkaralkenyl, Aralkinyl oder Alkaralkinyl bedeuten, die gegebenenfalls ein- oder mehrfach durch $(C_6H_5)_2P$-, $(R^5O)_2P(O)$-, $R^5_3$ Si-, $R^5_3$ SiO-, $R^5SO_2$-, -S-$C_2$-$C_4$-Alkylen-S-, -O-$C_2$-$C_4$-Alkylen-O-, wobei $R^5$ für Phenyl, Benzyl oder $C_1$-$C_8$-Alkyl steht, Cyano, F, Nitro, $C_1$-$C_{12}$-Alkylthio, $C_1$-$C_{12}$-Alkoxy, Sekundäramino oder -$COR^4$, worin $R^4$ der Rest eines einwertigen Alkohols ist, substituiert sind; oder einen Rest eines Enols, Enamins oder Enhydrazins bedeutet;
$R^2$ gegebenenfalls durch Alkyl, Alkenyl, Alkoxy, Cycloalkyl, Aryl, Aralkyl, Trialkoxysilyl, Trialkylsilyl oder Halogen substituiertes Cyclopentadienyl, Alkoxy, Cycloalkoxy, Cycloalkylalkoxy, Aryloxy, Aralkyloxy, Alkylthio, Arylthio, Aralkylthio; Halogen, Pseudohalogen, Acyloxy, Acylamino oder Trialkylsilyloxy darstellt,
$R^3$ der um ein Hydroxyl-, Thiol- oder Aminwasserstoffatom verminderte Rest eines geschützten, monohydroxyfunktionellen, monothiolfunktionellen oder monoaminfunktionellen, optisch aktiven Zuckers, Thiozuckers oder Aminozuckers, oder deren Derivaten aus der Gruppe der Zuckeralkohole; Ester einer Zuckersäure, Aldozuckersäure oder Ketozuckersäure; Aminozucker,
Zuckermercaptale oder Desoxyzucker ist,
x für 0, 1 oder 2 und y für 0, 1, 2 oder 3 und
$M^⊕$ für $Li^⊕$, $Na^⊕$, $K^⊕$, $MgY^⊕$, $ZnY^⊕$, $CdY^⊕$, $HgY^⊕$, $CuY^⊕$ oder quaternäres Ammonium stehen, worin Y Halogen bedeutet, dadurch gekennzeichnet, dass man eine Verbindung der Formel II

$$R^2_x R^3_{3-x} MeX \qquad (II)$$

oder eine Verbindung der Formel (IIa)

$$R^2_y R^3_{4-y} Me \qquad (IIa)$$

in Gegenwart eines inerten Lösungsmittels und eines inerten Schutzgases mit einer Verbindung der Formel III

$R^{1\ominus}M^{\oplus}$ (III)

umsetzt, wobei Me, $R^1$, $R^2$, $R^3$, $M^{\oplus}$, x und y die zuvor angegebenen Bedeutungen haben und X für ein Anion steht.

**2.** Verfahren gemäss Anspruch 1, worin $R^1$ gegebenenfalls ein- oder mehrfach durch Sekundäramino, Cyano, Nitro, $C_1$-$C_6$-Alkylthio, $C_1$-$C_6$-Alkoxy oder -$COR^4$, worin $R^4$ $C_1$-$C_{12}$-Alkoxy ist, substituiertes lineares oder verzweigtes $C_1$-$C_{18}$-Alkyl, C-$_2$-$C_{12}$-Alkenyl, $C_2$-$C_{12}$-Alkinyl, Cycloalkyl mit 3-8 Ring-C-Atomen, Cycloalkenyl mit 3 bis 8 Ring-C-Atomen, $C_6$-$C_{12}$-Aryl, $C_7$-$C_{16}$-Alkaryl oder Aralkyl, $C_8$-$C_{16}$-Alkaralkyl, $C_8$-$C_{16}$-Aralkenyl, $C_9$-$C_{16}$-Alkaralkenyl,$C_8$-$C_{16}$-Aralkinyl, $C_9$-$C_{16}$-Alkaralkinyl; oder ein über das Enolsauerstoffatom oder über das Enaminstickstoffatom gebundener Rest eine Enols, Enamins oder Enhydrazins.

**3.** Verfahren gemäss Anspruch 2, worin $R^1$ gegebenenfalls ein- oder mehrfach durch Sekundäramino, Cyano, Nitro, $C_1$-$C_6$-Alkoxy, $C_1$-$C_6$-Alkylthio oder -$COR^4$, worin $R^4$ $C_1$-$C_{12}$-Alkoxy ist, substituiertes lineares oder verzweigtes $C_1$-$C_6$-Alkyl, $C_2$-$C_6$-Alkenyl, $C_2$-$C_6$-Alkinyl, Cycloalkyl mit 3-6 Ring-C-Atomen, Cycloalkenyl mit 3 bis 6 Ring-C-Atomen, Phenyl, ($C_1$-$C_{10}$-Alkyl)phenyl, Phenyl($C_1$-$C_2$-alkyl), ($C_1$-$C_8$-Alkyl)phenyl($C_1$-$C_2$-alkyl), Phenylvinyl, Phenylethinyl oder Phenylpropargyl, ($C_1$-$C_8$-Alkyl)phenylvinyl, ($C_1$-$C_8$-Alkyl)phenylethinyl oder ($C_1$-$C_7$-Alkyl)phenylpropargyl; oder ein über ein Enolsauerstoffatom oder Enaminostickstoffatom gebundener Rest eines Enols, Enamins oder Enhydrazins mit bis zu 20 C-Atomen.

**4.** Verfahren gemäss Anspruch 2, worin $R^1$ gegebenenfalls ein- oder mehrfach mit Sekundäramino, Cyano, Nitro, $C_1$-$C_6$-Alkoxy, $C_1$-$C_6$-Alkylthio oder -$COR^4$, worin $R^4$ $C_1$-$C_{12}$-Alkoxy ist, substituiertes lineares oder verzweigtes $C_1$-$C_4$-Alkyl, Vinyl, Allyl, Ethinyl, Propargyl, Cyclopentyl, Cyclohexyl, Cyclopentenyl und Cyclohexenyl, Phenyl, Methylphenyl, Benzyl, 1-Phenyleth-2-yl, Methylbenzyl, Phenylvinyl, Methylphenylvinyl, Phenylethinyl, Phenylpropargyl, Methylphenylethinyl, Dimethylphenylethinyl oder -propargyl; oder ein über das Enolsauerstoffatom oder über das Enaminostickstoffatom gebundener Rest eines Enols, Enamins oder Enhydrazins mit 2-16 C-Atomen.

**5.** Verfahren gemäss Anspruch 1, worin $R^2$ gegebenenfalls durch $C_1$-$C_6$-Alkyl, $C_2$-$C_6$-Alkenyl, $C_1$-$C_6$-Alkoxy, Cycloalkyl mit 5 oder 6 Ring-C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, oder Br substituiertes Cyclopentadienyl, $C_1$-$C_{18}$-Alkoxy, $C_6$-$C_{12}$-Aryloxy, $C_7$-$C_{16}$-Aralkyloxy, $C_1$-$C_{18}$-Alkylthio, $C_6$-$C_{12}$-Arylthio, $C_7$-$C_{16}$-Aralkylthio; Halogen, Pseudohalogen, $C_1$-$C_{18}$-Acyloxy, $C_1$-$C_{18}$-Acylamino oder Trialkylsilyloxy mit 1 bis 6 C-Atomen in den Alkylgruppen darstellt.

**6.** Verfahren gemäss Anspruch 5, worin $R^2$ gegebenenfalls wie in Anspruch 5 angegeben substituiertes Cyclopentadienyl, $C_1$-$C_6$-Alkoxy, Phenoxy, Benzyloxy, $C_1$-$C_6$-Alkylthio, Phenylthio, Benzylthio; Cl, Br, J, CN, CNS, CNO, $C_1$-$C_{12}$-Acyloxy, $C_1$-$C_{12}$-Acylamino oder Trialkylsilyloxy mit 1 bis 4 C-Atomen in den Alkylgruppen darstellt.

**7.** Verfahren gemäss Anspruch 1, worin $R^3$ der Rest eines geschützten monohydroxy-, monothiol- oder monoaminofunktionellen $C_3$-$C_7$-Monosaccharids oder entsprechender Di- oder Trisaccharide oder deren

Derivaten aus der Gruppe der Zuckeralkohole; Ester einer Zuckersäure, Aldozuckersäure oder Ketozukkersäure; Aminozucker, Desoxyzucker oder Zuckermercaptale ist.

**8.** Verfahren gemäss Anspruch 7, worin $R^3$ den Rest eines $C_5$- oder $C_6$-Monosaccharids oder dessen Derivate bedeutet.

**9.** Verfahren gemäss Anspruch 7, worin $R^3$ der Rest einer geschützten Furanose oder Pyranose ist.

**10.** Verfahren gemäss Anspruch 1, worin die Hydroxylgruppen der Zucker und deren Derivate durch $C_1$-$C_8$-Acyl, $C_1$-$C_8$-Alkyl, Benzyl, Diphenylmethyl, Trityl, $C_1$-$C_8$-Alkyliden, Triphenylsilyl oder Trialkylsilyl mit 1 bis 8 C-Atomen in den Alkylgruppen, $(C_6H_5)_2Si=$, $(C_1\text{-}C_8\text{-Alkyl})_2Si$, $(C_6H_5)_2Sn=$ oder $(C_1\text{-}C_8\text{-Alkyl})_2Sn=$ geschützt sind.

**11.** Verfahren gemäss Anspruch 1, worin $R^3$ der um das Hydroxylwasserstoffatom verminderte Rest von 1,2:5,6-Di-O-isopropyliden-α-glucofuranoseist.

**12.** Verfahren gemäss Anspruch 1, worin x für 1 und y für 1 stehen.

**13.** Verfahren gemäss Anspruch 1, worin Y Cl, Br oder J bedeuten.

**14.** Verfahren gemäss Anspruch 1, worin $M^{\oplus}$ $Li^{\oplus}$, $MgCl^{\oplus}$, $MgBr^{\oplus}$, $ZnCl^{\oplus}$, $ZnBr^{\oplus}$, $CdCl^{\oplus}$, $CdBr^{\oplus}$ oder Tetraalkylammonium mit 1 bis 6 C-Atomen in den Alkylgruppen bedeutet.

**15.** Verfahren gemäss Anspruch 1, worin Me für Ti steht, $R^1$ Allyl oder 1-(t-Butyloxy)-vinyl-1-oxyl bedeuten, $R^2$ Cyclopentadienyl ist und $R^3$ der um das Hydroxylwasserstoffatom verminderte Rest von 1,2:5,6-Di-O-isopropyliden-α-glucofuranose ist.

**16.** Verfahren zur Herstellung von Verbindungen der Formel II

$$R^2_xR^3_{3-x}MeX \quad (II)$$

worin Me, $R^2$, $R^3$ und x die in Anspruch 1 angegebene Bedeutung haben, dadurch gekennzeichnet, dass man 1 Mol eines Salzes der Formel

$R^2_xMeX_{4-y}$

mit 1 bis 3 mol eines optisch aktiven, geschützten monohydroxy-, monothiol- oder monoaminofunktionellen Zuckers, Thiozuckers oder Aminozuckers oder deren Derivaten aus der Gruppe der Zuckeralkohole; Esters einer Zucker-, Aldo- oder Ketozuckersäure; Aminozucker, Zuckermercaptale oder Desoxyzucker der formel $R^3H$ umsetzt.

**17.** Verwendung von Verbindungen der Formeln I und Ia gemäss Anspruch 1 als enantioselektive Reaktanden für Verbindungen mit Carbonyl- und/oder N-substituierten Imingruppen.

**18.** Verfahren zur Herstellung von sekundären und tertiären Alkoholen und sekundären Aminen, dadurch gekennzeichnet, dass man Aldehyde, Ketone, N-substituierte Aldimine oder Ketimine mit 1 Mol einer Verbindung der Formel I oder Ia gemäss Anspruch 1 pro Mol Aldehyd-, Keto- oder Imingruppe umsetzt.

**19.** Verfahren gemäss Anspruch 18, dadurch gekennzeichnet, dass es in Gegenwart eines inerten Lösungsmittels und von inerten Schutzgasen durchgeführt wird.

**20.** Verfahren gemäss Anspruch 19, dadurch gekennzeichnet, dass es bei Temperaturen von -80 bis 30 °C durchgeführt wird.

## Claims

**Claims for the following Contracting States : BE, CH, DE, FR, GB, IT, LI, LU, NL, SE**

**1.** A compound of the formula I or Ia

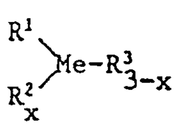 (I) or 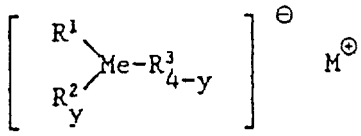 (Ia),

in which
Me is tetravalent titanium, zirconium or hafnium,
$R^1$ is linear or branched alkyl, alkenyl or alkynyl, cycloalkyl or cycloalkenyl each of which is unsubstituted or substituted by $C_1$-$C_4$alkyl, or is aryl, alkaryl, aralkyl, alkaralkyl, aralkenyl, alkaralkenyl, aralkynyl or alkaralkynyl which are unsubstituted or monosubstituted or polysubstituted by $(C_6H_5)_2P$-, $(R^5O)_2P(O)$-, $R^5_3$ Si-, $R^5_3$ SiO-, $R^5SO_2$-, -S-$C_2$-$C_4$alkylene-S-, -O-$C_2$-$C_4$alkylene-O-, $R^5$ being phenyl, benzyl or $C_1$-$C_8$alkyl, or is cyano, F, nitro, $C_1$-$C_{12}$alkylthio, $C_1$-$C_{12}$alkoxy, secondary amino or -$COR^4$ in which $R^4$ is the radical of a monohydric alcohol; or $R^1$ is a radical of an enol, enamine or enehydrazine;
$R^2$ is cyclopentadienyl, alkoxy, cycloalkoxy, cycloalkylalkoxy, aryloxy, aralkyloxy, alkylthio, arylthio or aralkylthio each of which is unsubstituted or substituted by alkyl, alkenyl, alkoxy, cycloalkyl, aryl, aralkyl, trialkoxysilyl, trialkylsilyl or halogen, or is halogen, pseudohalogen, acyloxy, acylamino or trialkylsilyloxy,
$R^3$ is the radical, diminished by a hydroxyl, thiol or amine hydrogen atom, of a protected, monohydroxy-functional, monothiol-functional or monoamine-functional, optically active sugar, thio-sugar or amino-sugar or derivatives thereof belonging to the group of sugar alcohols; esters of a sugar acid, aldo-sugar acid or keto-sugar acid; amino-sugars, sugar mercaptals or deoxy-sugars,
x is 0, 1 or 2 and y is 0, 1, 2 or 3 and
$M^{\oplus}$ is $Li^{\oplus}$, $Na^{\oplus}$, $K^{\oplus}$, $MgY^{\oplus}$, $ZnY^{\oplus}$, $CdY^{\oplus}$, $HgY^{\oplus}$, $CuY^{\oplus}$ or quaternary ammonium, Y being halogen.

**2.** A compound according to claim 1, in which $R^1$ is linear or branched $C_1$-$C_{18}$alkyl, $C_2$-$C_{12}$alkenyl, $C_2$-$C_{12}$alkynyl, cycloalkyl having 3-8 ring carbon atoms, cycloalkenyl having 3 to 8 ring carbon atoms, $C_6$-$C_{12}$aryl, $C_7$-$C_{16}$alkaryl or aralkyl, $C_8$-$C_{16}$alkaralkyl, $C_8$-$C_{16}$aralkenyl, $C_9$-$C_{16}$alkaralkenyl, $C_8$-$C_{16}$aralkynyl or $C_9$-$C_{16}$alkaralkynyl each of which is unsubstituted or monosubstituted or polysubstituted by secondary amino, cyano, nitro, $C_1$-$C_6$alkylthio, $C_1$-$C_6$alkoxy or -$COR^4$ in which $R^4$ is $C_1$-$C_{12}$alkoxy; or $R^1$ is a radical of an enol, enamine or enehydrazine which is attached via the enol oxygen atom or via the enamine nitrogen atom.

**3.** A compound according to claim 2, in which $R^1$ is linear or branched $C_1$-$C_6$alkyl, $C_2$-$C_6$alkenyl, $C_2$-$C_6$alkynyl, cycloalkyl having 3-6 ring carbon atoms, cycloalkenyl having 3 to 6 ring carbon atoms, phenyl, ($C_1$-$C_{10}$alkyl)phenyl, phenyl($C_1$-$C_2$alkyl), ($C_1$-$C_8$alkyl)phenyl($C_1$-$C_2$alkyl), phenylvinyl, phenylethynyl, phenylpropargyl, ($C_1$-$C_8$alkyl)phenylvinyl, ($C_1$-$C_8$alkyl)phenylethynyl or ($C_1$-$C_7$alkyl)-phenylpropargyl each of which is unsubstituted or monosubstituted or polysubstituted by secondary amino, cyano, nitro, $C_1$-$C_6$alkoxy, $C_1$-$C_6$alkylthio or -$COR^4$ in which $R^4$ is $C_1$-$C_{12}$alkoxy; or $R^1$ is the radical of an enol, enamine or enehydrazine having up to 20 carbon atoms which is attached via an enol oxygen atom or enamine nitrogen atom.

**4.** A compound according to claim 2, in which $R^1$ is linear or branched $C_1$-$C_4$alkyl, vinyl, allyl, ethynyl, propargyl, cyclopentyl, cyclohexyl, cyclopentenyl, cyclohexenyl, phenyl, methylphenyl, benzyl, 1-phenyleth-2-yl, methylbenzyl, phenylvinyl, methylphenylvinyl, phenylethynyl, phenylpropargyl, methylphenylethynyl, dimethylphenylethynyl or dimethylphenylpropargyl each of which is unsubstituted or monosubstituted or polysubstituted by secondary amino, cyano, nitro, $C_1$-$C_6$alkoxy, $C_1$-$C_6$alkylthio or -$COR^4$ in which $R^4$ is $C_1$-$C_{12}$alkoxy; or $R^1$ is the radical of an enol, enamine or enehydrazine having 2-16 carbon atoms which is attached via the enol oxygen atom or via the enamine nitrogen atom.

**5.** A compound according to claim 1, in which $R^2$ is cyclopentadienyl, $C_1$-$C_{18}$alkoxy, $C_6$-$C_{12}$aryloxy, $C_7$-$C_{16}$aralkoxy, $C_1$-$C_{18}$alkylthio, $C_6$-$C_{12}$arylthio or $C_7$-$C_{16}$aralkylthio each of which is unsubstituted or

substituted by $C_1$-$C_6$alkyl, $C_2$-$C_6$alkenyl, $C_1$-$C_6$alkoxy, cycloalkyl having 5 or 6 ring carbon atoms, phenyl, benzyl, trialkoxysilyl having 1 to 6 carbon atoms in the alkoxy groups, trialkylsilyl having 1 to 6 carbon atoms in the alkyl groups, F, Cl or Br, or is halogen, pseudohalogen, $C_1$-$C_{18}$acyloxy, $C_1$-$C_{18}$acylamino or trialkylsilyloxy having 1 to 6 carbon atoms in the alkyl groups.

**6.** A compound according to claim 5, in which $R^2$ is cyclopentadienyl, $C_1$-$C_6$alkoxy, phenoxy, benzyloxy, $C_1$-$C_6$alkylthio, phenylthio or benzylthio each of which is unsubstituted or substituted as stated in claim 5; or is Cl, Br, I, CN, CNS, CNO, $C_1$-$C_{12}$acyloxy, $C_1$-$C_{12}$acylamino or trialkylsilyloxy having 1 to 4 carbon atoms in the alkyl groups.

**7.** A compound according to claim 1, in which $R^3$ is the radical of a protected, monohydroxy-functional, monothiol-functional or monoamino-functional $C_3$-$C_7$ monosaccharide or corresponding disaccharides or trisaccharides or derivatives thereof belonging to the group of sugar alcohols; esters of a sugar acid, aldo-sugar acid or keto-sugar acid; amino-sugars, deoxy-sugars or sugar mercaptals.

**8.** A compound according to claim 7, in which $R^3$ is the radical of a $C_5$ or $C_6$ monosaccharide or derivatives thereof.

**9.** A compound according to claim 7, in which $R^3$ is the radical of a protected furanose or pyranose.

**10.** A compound according to claim 1, in which the hydroxyl groups of the sugars and their derivatives are protected by $C_1$-$C_8$acyl, $C_1$-$C_8$alkyl, benzyl, diphenylmethyl, trityl, $C_1$-$C_8$alkylidene, triphenylsilyl or trialkylsilyl having 1 to 8 carbon atoms in the alkyl groups, $(C_6H_5)_2Si=$, $(C_1$-$C_8alkyl)_2Si$, $(C_6H_5)_2Sn=$ or $(C_1$-$C_8alkyl)_2Sn=$.

**11.** A compound according to claim 1, in which $R^3$ is the radical of 1,2:5,6-di-O-isopropylidene-α-glucofuranose which has been diminished by the hydroxyl hydrogen atom.

**12.** A compound according to claim 1, in which x is 1 and y is 1.

**13.** A compound according to claim 1, in which Y is Cl, Br or I.

**14.** A compound according to claim 1, in which $M^{\oplus}$ is $Li^{\oplus}$, $MgCl^{\oplus}$, $MgBr^{\oplus}$, $ZnCl^{\oplus}$, $ZnBr^{\oplus}$, $CdCl^{\oplus}$, $CdBr^{\oplus}$, or tetraalkylammonium having 1 to 6 carbon atoms in the alkyl groups.

**15.** A compound according to claim 1, in which Me is Ti, $R^1$ is allyl or 1-(tert-butoxy)vinyl-l-oxyl, $R^2$ is cyclopentadienyl and $R^3$ is the radical of 1,2:5,6-di-O-isopropylidene-α-glucofuranose which has been diminished by the hydroxyl hydrogen atom.

**16.** A process for the preparation of a compound of the formula I or Ia according to claim 1, which comprises reacting, in the presence of an inert solvent and an inert protective gas, a compound of the formula II

$$R^2_xR^3_{3-x}MeX \qquad (II)$$

or a compound of the formula IIa

$$R^2_yR^3_{4-y}Me \qquad (IIa)$$

with a compound of the formula III

$R^{1\ominus}M^{\oplus}$ (III)

in which Me, $R^1$, $R^2$, $R^3$, $M^{\oplus}$, x and y are as defined in claim 1 and X is an anion.

**17.** A compound of the formula II

$$R^2_x R^3_{3-x} MeX \qquad (II)$$

in which Me, $R^2$, $R^3$ and x are as defined in claim 1.

**18.** The use of a compound of the formula I or Ia according to claim 1 as enantioselective reactants for compounds containing carbonyl and/or N-substituted imine groups.

**19.** A process for the preparation of secondary and tertiary alcohols and secondary amines, which comprises reacting aldehydes, ketones, N-substituted aldimines or ketimines with 1 mol of a compound of the formula I or Ia according to claim 1 per mole of aldehyde, keto or imine group.

**20.** A process according to claim 19, which is carried out at temperatures from -80 to 30 °C.

**Claims for the following Contracting States : AT, GR, ES**

**1.** A process for the preparation of a compound of the formula I or Ia

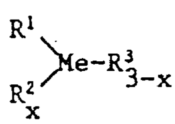 (I) or 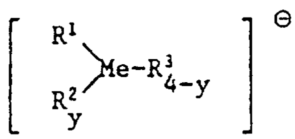 $M^{\oplus}$ (Ia),

in which
Me is tetravalent titanium, zirconium or hafnium,
$R^1$ is linear or branched alkyl, alkenyl, alkynyl, cycloalkyl or cycloalkenyl each of which is unsubstituted or substituted by $C_1$-$C_4$alkyl, or is aryl, alkaryl, aralkyl, alkaralkyl, aralkenyl, alkaralkenyl, aralkynyl or alkaralkynyl which are unsubstituted or monosubstituted or polysubstituted by $(C_6H_5)_2P$-, $(R^5O)_2P(O)$-, $R^5_3$ Si-, $R^5_3$ SiO-, $R^5SO_2$-, -S-$C_2$-$C_4$alkylene-S-, -O-$C_2$-$C_4$alkylene-O-, $R^5$ being phenyl, benzyl or $C_1$-$C_8$alkyl, or is cyano, F, nitro, $C_1$-$C_{12}$alkylthio, $C_1$-$C_{12}$alkoxy, secondary amino or -$COR^4$ in which $R^4$ is the radical of a monohydric alcohol; or $R^1$ is a radical of an enol, enamine or enehydrazine;
$R^2$ is cyclopentadienyl, alkoxy, cycloalkoxy, cycloalkylalkoxy, aryloxy, aralkyloxy, alkylthio, arylthio or aralkylthio each of which is unsubstituted or substituted by alkyl, alkenyl, alkoxy, cycloalkyl, aryl, aralkyl, trialkoxysilyl, trialkylsilyl or halogen, or is halogen, pseudohalogen, acyloxy, acylamino or trialkylsilyloxy,
$R^3$ is the radical, diminished by a hydroxyl, thiol or amine hydrogen atom, of a protected, monohydroxy-functional, monothiol-functional or monoamine-functional, optically active sugar, thio-sugar or amino-sugar or derivatives thereof belonging to the group of sugar alcohols; esters of a sugar acid, aldo-sugar acid or keto-sugar acid; amino-sugars, sugar mercaptals or deoxy-sugars,
x is 0, 1 or 2 and y is 0, 1, 2 or 3 and
$M^{\oplus}$ is $Li^{\oplus}$, $Na^{\oplus}$, $K^{\oplus}$, $MgY^{\oplus}$, $ZnY^{\oplus}$, $CdY^{\oplus}$, $HgY^{\oplus}$, $CuY^{\oplus}$ or quaternary ammonium, Y being halogen, which comprises reacting, in the presence of an inert solvent and an inert protective gas, a compound of the formula II

$$R^2_x R^3_{3-x} MeX \qquad (II)$$

or a compound of the formula IIa

$$R^2_y R^3_{4-y} Me \qquad (IIa)$$

with a compound of the formula III

$R^{1\ominus}M^{\oplus}$ (III)

in which Me, $R^1$, $R^2$, $R^3$, $M^{\oplus}$, x and y are as defined above and X is an anion.

**2.** A process according to claim 1, in which $R^1$ is linear or branched $C_1$-$C_{18}$alkyl, $C_2$-$C_{12}$alkenyl, $C_2$-$C_{12}$alkynyl, cycloalkyl having 3-8 ring carbon atoms, cycloalkenyl having 3 to 8 ring carbon atoms, $C_6$-$C_{12}$aryl, $C_7$-$C_{16}$alkaryl or aralkyl, $C_8$-$C_{16}$alkaralkyl, $C_8$-$C_{16}$aralkenyl, $C_9$-$C_{16}$alkaralkenyl, $C_8$-$C_{16}$aralkynyl or $C_9$-$C_{16}$alkaralkynyl each of which is unsubstituted or monosubstituted or polysubstituted by secondary amino, cyano, nitro, $C_1$-$C_6$alkylthio, $C_1$-$C_6$alkoxy or -$COR^4$ in which $R^4$ is $C_1$-$C_{12}$alkoxy; or R' is a radical of an enol, enamine or enehydrazine which is attached via the enol oxygen atom or via the enamine nitrogen atom.

**3.** A process according to claim 2, in which $R^1$ is linear or branched $C_1$-$C_6$alkyl, $C_2$-$C_6$alkenyl, $C_2$-$C_6$alkynyl, cycloalkyl having 3-6 ring carbon atoms, cycloalkenyl having 3 to 6 ring carbon atoms, phenyl, ($C_1$-$C_{10}$alkyl)phenyl, phenyl($C_1$-$C_2$alkyl), ($C_1$-$C_8$alkyl)phenyl($C_1$-$C_2$alkyl), phenylvinyl, phenylethynyl, phenylpropargyl, ($C_1$-$C_8$alkyl)phenylvinyl, ($C_1$-$C_8$alkyl)phenylethynyl or ($C_1$-$C_7$alkyl)-phenylpropargyl each of which is unsubstituted or monosubstituted or polysubstituted by secondary amino, cyano, nitro, $C_1$-$C_6$alkoxy, $C_1$-$C_6$alkylthio or -$COR^4$ in which $R^4$ is $C_1$-$C_{12}$alkoxy; or $R^1$ is the radical of an enol, enamine or enehydrazine having up to 20 carbon atoms which is attached via an enol oxygen atom or enamine nitrogen atom.

**4.** A process according to claim 2, in which $R^1$ is linear or branched $C_1$-$C_4$alkyl, vinyl, allyl, ethynyl, propargyl, cyclopentyl, cyclohexyl, cyclopentenyl, cyclohexenyl, phenyl, methylphenyl, benzyl, 1-phenyleth-2-yl, methylbenzyl, phenylvinyl, methylphenylvinyl, phenylethynyl, phenylpropargyl, methylphenylethynyl, dimethylphenylethynyl or dimethylphenylpropargyl each of which is unsubstituted or monosubstituted or polysubstituted by secondary amino, cyano, nitro, $C_1$-$C_6$alkoxy, $C_1$-$C_6$alkylthio or -$COR^4$ in which $R^4$ is $C_1$-$C_{12}$alkoxy; or $R^1$ is the radical of an enol, enamine or enehydrazine having 2-16 carbon atoms which is attached via the enol oxygen atom or via the enamine nitrogen atom.

**5.** A process according to claim 1, in which $R^2$ is cyclopentadienyl, $C_1$-$C_{18}$alkoxy, $C_6$-$C_{12}$aryloxy, $C_7$-$C_{16}$aralkoxy, $C_1$-$C_{18}$alkylthio, $C_6$-$C_{12}$arylthio or $C_7$-$C_{16}$aralkylthio each of which is unsubstituted or substituted by $C_1$-$C_6$alkyl, $C_2$-$C_6$alkenyl, $C_1$-$C_6$alkoxy, cycloalkyl having 5 or 6 ring carbon atoms, phenyl, benzyl, trialkoxysilyl having 1 to 6 carbon atoms in the alkoxy groups, trialkylsilyl having 1 to 6 carbon atoms in the alkyl groups, F, Cl or Br, or is halogen, pseudohalogen, $C_1$-$C_{18}$acyloxy, $C_1$-$C_{18}$acylamino or trialkylsilyloxy having 1 to 6 carbon atoms in the alkyl groups.

**6.** A process according to claim 5, in which $R^2$ is cyclopentadienyl, $C_1$-$C_6$alkoxy, phenoxy, benzyloxy, $C_1$-$C_6$alkylthio, phenylthio or benzylthio each of which is unsubstituted or substituted as stated in claim 5; or is Cl, Br, I, CN, CNS, CNO, $C_1$-$C_{12}$acyloxy, $C_1$-$C_{12}$acylamino or trialkylsilyloxy having 1 to 4 carbon atoms in the alkyl groups.

**7.** A process according to claim 1, in which $R^3$ is the radical of a protected, monohydroxy-functional, monothiol-functional or monoamino-functional $C_3$-$C_7$ monosaccharide or corresponding disaccharides or trisaccharides or derivatives thereof belonging to the group of sugar alcohols; esters of a sugar acid, aldo-sugar acid or keto-sugar acid; amino-sugars, deoxy-sugars or sugar mercaptals.

**8.** A process according to claim 7, in which $R^3$ is the radical of a $C_5$ or $C_6$ monosaccharide or derivatives thereof.

**9.** A process according to claim 7, in which $R^3$ is the radical of a protected furanose or pyranose.

**10.** A process according to claim 1, in which the hydroxyl groups of the sugars and their derivatives are protected by $C_1$-$C_8$acyl, $C_1$-$C_8$alkyl, benzyl, diphenylmethyl, trityl, $C_1$-$C_8$alkylidene, triphenylsilyl or trialkylsilyl having 1 to 8 carbon atoms in the alkyl groups, $(C_6H_5)_2Si=$, $(C_1$-$C_8$alkyl$)_2Si$, $(C_6H_5)_2Sn=$ or $(C_1$-$C_8$alkyl$)_2Sn=$.

**11.** A process according to claim 1, in which $R^3$ is the radical of 1,2:5,6-di-O-isopropylidene-$\alpha$-glucofuranose which has been diminished by the hydroxyl hydrogen atom.

**12.** A process according to claim 1, in which x is 1 and y is 1.

**13.** A process according to claim 1, in which Y is Cl, Br or I.

**14.** A process according to claim 1, in which $M^{\oplus}$ is $Li^{\oplus}$, $MgCl^{\oplus}$, $MgBr^{\oplus}$, $ZnCl^{\oplus}$, $ZnBr^{\oplus}$, $CdCl^{\oplus}$, $CdBr^{\oplus}$, or tetraalkylammonium having 1 to 6 carbon atoms in the alkyl groups.

**15.** A process according to claim 1, in which Me is Ti, $R^1$ is allyl or 1-(tert-butoxy)vinyl-1-oxyl, $R^2$ is cyclopentadienyl and $R^3$ is the radical of 1,2:5,6-di-O-isopropylidene-$\alpha$-glucofuranose which has been diminished by the hydroxyl hydrogen atom.

**16.** A process for the preparation of a compound of the formula II

$$R^2_xR^3_{3-x}MeX \qquad (II)$$

in which Me, $R^2$, $R^3$ and x are as defined in claim 1, which comprises reacting 1 mol of a salt of the formula

$$R^2_x MeX_{4-y}$$

with 1 to 3 mol of an optically active protected, monohydroxy-functional, monothiol-functional or monoamino-functional sugar, thio-sugar or amino-sugar or derivatives thereof belonging to the group of sugar alcohols; esters of a sugar acid, aldo-sugar acid or keto-sugar acid; amino sugars, sugar mercaptals or deoxy-sugars of the formula $R^3H$.

**17.** The use of a compound of the formula I or Ia according to claim 1 as enantioselective reactants for compounds containing carbonyl and/or N-substituted imine groups.

**18.** A process for the preparation of secondary and tertiary alcohols and secondary amines, which comprises reacting aldehydes, ketones, N-substituted aldimines or ketimines with 1 mol of a compound of the formula I or Ia according to claim 1 per mole of aldehyde, keto or imine group.

**19.** A process according to claim 18, which is carried out in the presence of an inert solvent and inert protective gases.

**20.** A process according to claim 19, which is carried out at temperatures from -80 to 30 °C.

## Revendications

**Revendications pour les Etats contractants suivants : BE, CH, DE, FR, GB, IT, LI, LU, NL, SE**

**1.** Composés de formules I et Ia :

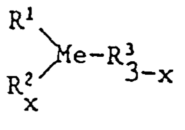 (I) et 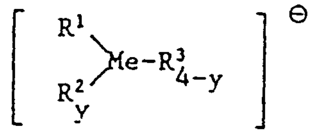 $M^{\oplus}$ (Ia),

dans lesquelles :
Me représente un atome de titane, de zirconium ou d'hafnium tétravalent ;
$R^1$ représente un groupe alkyle, alcényle ou alcynyle linéaire ou ramifié, un groupe cycloalkyle ou cycloalcényle éventuellement substitué par un ou des groupes alkyle en $C_1$-$C_4$, ou représente un groupe aryle, alkylaryle, aralkyle, alkylaralkyle, aralcényle, alkylaralcényle, aralcynyle ou alkylaralcynyle, tous groupes qui sont éventuellement substitués une ou plusieurs fois par des groupes : $(C_6H_5)_2P$-, $(R^5O)_2P(O)$-, $R^5_3$ Si-, $R^5_3$ SiO-, $R^5SO_2$-, -S-(alkylène en $C_2$-$C_4$)-S-, -O-(alkylène en $C_2$-$C_4$)-O-, où $R^5$ représente un groupe phényle, benzyle ou alkyle en $C_1$-$C_8$, ou par un groupe cyano, F, nitro, alkylthio en $C_1$-$C_{12}$, alcoxy en $C_1$-$C_{12}$, amino secondaire ou -$COR^4$, où $R^4$ représente le reste d'un alcool monovalent ; ou représente un reste d'un énol, d'une énamine ou d'une énhydrazine ;
$R^2$ représente un groupe cyclopentadiényle, alcoxy, cycloalcoxy, cycloalkylalcoxy, aryloxy, aralkyloxy, alkylthio, arylthio ou aralkylthio éventuellement substitué par un ou des groupes alkyle, alcényle, alcoxy, cycloalkyle, aryle, aralkyle, trialcoxysilyle, trialkylsilyle ou des atomes d'halogènes ; ou représente un atome d'halogène, un pseudohalogène, un groupe acyloxy, acylamino ou trialkylsilyloxy,
$R^3$ représente le reste, diminué d'un atome d'hydrogène, d'un groupe hydroxyle, thiol ou amino, d'un sucre, thiosucre ou amino sucre optiquement actif, protégé, et présentant une fonctionnalité monohydroxy, monothiol ou monoamino, ou bien un tel reste de leurs dérivés du groupe des alcools de sucres ; ou un reste d'ester d'un acide saccharique, aldosaccharique, ou cétosaccharique ; un reste d'un aminosucre, d'un sucre-mercaptal ou d'un désoxysucre,
x vaut 0, 1 ou 2 et y vaut 0, 1, 2 ou 3, et
$M^{\oplus}$ représente $Li^{\oplus}$, $Na^{\oplus}$, $K^{\oplus}$, $MgY^{\oplus}$, $Zny^{\oplus}$, $CdY^{\oplus}$, $HgY^{\oplus}$, $CuY^{\oplus}$ ou représente un ammonium quaternaire, Y représentant un atome d'halogène.

**2.** Composés selon la revendication 1, dans lesquels $R^1$ représente un groupe alkylaralcynyle en $C_9$-$C_{16}$, aralcynyle en $C_8$-$C_{16}$, alkylaralcényle en $C_9$-$C_{16}$, aralcényle en $C_8$-$C_{16}$, alkylaralkyle en $C_8$-$C_{16}$, alkylaryle ou aralkyle en $C_7$-$C_{16}$, aryle en $C_6$-$C_{12}$, cycloalcényle avec de 3 à 8 atomes de carbone dans le cycle, cycloalkyle avec de 3 à 8 atomes de carbone dans le cycle, alcynyle en $C_2$-$C_{12}$, alcényle en $C_2$-$C_{12}$ ou alkyle en $C_1$-$C_{18}$, linéaire ou ramifié, éventuellement substitué une ou plusieurs fois par des groupes amino secondaire, cyano, nitro, alkylthio en $C_1$-$C_6$, alcoxy en $C_1$-$C_6$ ou -$COR^4$, où $R^4$ représente un groupe alcoxy en $C_1$-$C_{12}$ ; ou représente un reste d'un énol, d'une énamine ou d'une énhydrazine fixé par l'atome d'oxygène de l'énol ou l'atome d'azote de l'énamine.

**3.** Composés selon la revendication 2, dans lesquels $R^1$ représente un groupe (alkyle en $C_1$-$C_7$)-phénylpropargyle, ou (alkyle en $C_1$-$C_8$)phényléthynyle, (alkyle en $C_1$-$C_8$)phénylvinyle, phénylvinyle, phényléthynyle, ou phénylpropargyle, (alkyle en $C_1$-$C_8$)phényl(alkyle en $C_1$-$C_2$), phényl(alkyle en $C_1$-$C_2$), (alkyle en $C_1$-$C_{10}$)phényle, phényle, cycloalcényle avec de 3 à 6 atomes de carbone dans le cycle, cycloalkyle avec de 3 à 6 atomes de carbone dans le cycle, alcynyle en $C_2$-$C_6$, alcényle en $C_2$-$C_6$, alkyle en $C_1$-$C_6$, linéaire ou ramifié, éventuellement substitué une ou plusieurs fois par des groupes amino secondaire, cyano, nitro, alcoxy en $C_1$-$C_6$, alkylthio en $C_1$-$C_6$, ou -$COR^4$, où $R^4$ représente un groupe alcoxy en $C_1$-$C_{12}$ ; ou représente un reste d'un énol, d'une énamine ou d'une énhydrazine comportant jusqu'à 20 atomes de carbone, fixé par l'atome d'oxygène de l'énol ou par l'atome d'azote de l'énamine.

**4.** Composés selon la revendication 2, dans lesquels $R^1$ représente un groupe diméthylphényléthynyle ou -propargyle, méthylphényléthynyle, phénylpropargyle, phényléthynyle, méthylphénylvinyle, phénylvinyle, méthylbenzyle, 1-phényléthyl-2, benzyle, méthylphényle, phényle, cyclopentényle et cyclohexényle,

cyclohexyle, cyclopentyle, propargyle, éthynyle, allyle, vinyle, alkyle en $C_1$-$C_4$, linéaire ou ramifié, éventuellement substitué une ou plusieurs fois par des groupes amino secondaire, cyano, nitro, alcoxy en $C_1$-$C_6$, alkylthio en $C_1$-$C_6$ ou -$COR^4$, où $R^4$ représente un groupe alcoxy en $C_1$-$C_{12}$ ; ou représente un reste d'un énol, d'une énamine ou d'une énhydrazine comportant de 2 à 16 atomes de carbone, fixé par l'atome d'oxygène de l'énol ou par l'atome d'azote de l'énamine.

**5.** Composés selon la revendication 1, dans lesquels $R^2$ représente un groupe aralkylthio en $C_7$-$C_{16}$, arylthio en $C_6$-$C_{12}$, alkylthio en $C_1$-$C_{18}$, aralkyloxy en $C_7$-$C_{16}$, aryloxy en $C_6$-$C_{12}$, alcoxy en $C_1$-$C_{18}$, ou cyclopentadiényle, éventuellement substitué par des groupes alkyle en $C_1$-$C_6$, alcényle en $C_2$-$C_6$, alcoxy en $C_1$-$C_6$, cycloalkyle comportant 5 ou 6 atomes de carbone dans le cycle, phényle, benzyle, trialcoxysilyle comportant de 1 à 6 atomes de carbone dans les groupes alcoxy, trialkylsilyle comportant de 1 à 6 atomes de carbone dans les groupes alkyle, ou par des atomes de F, Cl ou Br ; ou représente un atome d'halogène, ou un groupe pseudo-halogène, acyloxy en $C_1$-$C_{18}$, acylamino en $C_1$-$C_{18}$ ou trialkylsilyloxy comportant de 1 à 6 atomes de carbone dans les groupes alkyle.

**6.** Composés selon la revendication 5, dans lesquels $R^2$ représente un groupe benzylthio, phénylthio alkylthio en $C_1$-$C_6$, benzyloxy, phénoxy, alcoxy en $C_1$-$C_6$ ou cyclopentadiényle, éventuellement substitué comme indiqué dans la revendication 5 ; ou représente un atome de Cl, Br, I, ou un groupe CN, CNS, CNO, acyloxy en $C_1$-$C_{12}$,acylamino en $C_1$-$C_{12}$ ou trialkylsilyloxy comportant de 1 à 4 atomes de carbone dans les groupes alkyle.

**7.** Composés selon la revendication 1, dans lesquels $R^3$ représente le reste d'un monosaccharide en $C_3$-$C_7$ présentant une fonction monohydroxy, monothiol ou monoamino, protégé, ou bien un tel reste de di- ou trisaccharides correspondants, ou de leurs dérivés du groupe des alcools de sucres ; ou d'un ester d'un acide saccharique, d'un acide aldosaccharique, ou d'un acide cétosaccharique ; ou d'un aminosucre, d'un désoxysucre, ou d'un sucre-mercaptal.

**8.** Composés selon la revendication 7, dans lesquels $R^3$ représente le reste d'un monosaccharide en $C_5$ ou $C_6$, ou d'un de ses dérivés.

**9.** Composés selon la revendication 7, dans lesquels $R^3$ représente le reste d'un pyranose ou furanose protégé.

**10.** Composés selon la revendication 1, dans lesquels les groupes hydroxy des sucres et de leurs dérivés sont protégés par des groupes acyle en $C_1$-$C_8$, alkyle en $C_1$-$C_8$, benzyle, diphénylméthyle, trityle, alkylidène en $C_1$-$C_8$,triphénylsilyle ou trialkylsilyle comportant de 1 à 8 atomes de carbone dans les groupes alkyle, ou $(C_6H_5)_2Si=$, (alkyle en $C_1$-$C_8)_2Si=$, $(C_6H_5)_2Sn=$ ou (alkyle en $C_1$-$C_8)_2Sn=$.

**11.** Composés selon la revendication 1, dans lesquels $R^3$ représente le reste, diminué de l'atome d'hydrogène du groupe hydroxyle, du 1,2:5,6-di-O-isopropylidène-$\alpha$-glucofuranose.

**12.** Composés selon la revendication 1, dans lesquels x vaut 1 et y vaut 1.

**13.** Composés selon la revendication 1, dans lesquels Y représente un atome de Cl, Br ou I.

**14.** Composés selon la revendication 1, dans lesquels $M^{\oplus}$ représente un ion $Li^{\oplus}$, $MgCl^{\oplus}$, $MgBr^{\oplus}$, $ZnCl^{\oplus}$, $ZnBr^{\oplus}$, $CdCl^{\oplus}$, $CdBr^{\oplus}$ ou tétraalkylammonium comportant de 1 à 6 atomes de carbone dans les groupes alkyle.

**15.** Composés selon la revendication 1, dans lesquels Me représente un atome de Ti, $R^1$ représente un groupe allyle ou un groupe 1-(t-butyloxy)-vinyl-1-oxyle, $R^2$ représente un groupe cyclopentadiényle, et $R^3$ représente le reste, diminué de l'atome d'hydrogène du groupe hydroxyle, du 1,2:5,6-di-O-isopropylidène-$\alpha$-glucofuranose.

**16.** Procédé pour la préparation des composés de formules I et Ia selon la revendication 1, caractérisé en ce que l'on fait réagir un composé de formule II :

$$R^2_x R^3_{3-x} MeX \qquad (II)$$

ou bien un composé de formule IIa :

$$R^2_y R^3_{4-y} Me \qquad (IIa)$$

en présence d'un solvant inerte et d'un gaz protecteur inerte, avec un composé de formule III :

$R^{1\ominus} M^{\oplus}$ (III),

Me, $R^1$, $R^2$, $R^3$, $M^{\oplus}$, x et y ayant les significations données dans la revendication 1, et X représentant un anion.

**17.** Composés de formule II :

$$R^2_x R^3_{3-x} MeX \qquad (II)$$

dans laquelle Me, $R^2$, $R^3$ et x sont tels que définis dans la revendication 1.

**18.** Utilisation des composés de formules I ou Ia selon la revendication 1 comme réactifs énantiosélectifs pour des composés comportant des groupes carbonyle et/ou imine N-substitués.

**19.** Procédé de préparation d'alcools secondaires et tertiaires et d'amines secondaires, caractérisé en ce que l'on fait réagir des aldéhydes, cétones, aldimines ou cétimines N-substituées avec 1 mole d'un composé de formule I ou Ia selon la revendication 1, par mole de groupe aldéhyde, céto ou imine.

**20.** Procédé selon la revendication 19, caractérisé en ce qu'il est mis en oeuvre à des températures de -80 à 30 °C.

**Revendications pour les Etats contractants suivants : AT, GR, ES**

**1.** Procédé pour la préparation de composés de formules I et Ia :

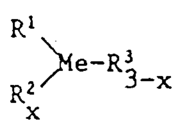 (I) et 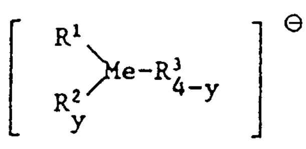 $M^{\oplus}$ (Ia),

dans lesquelles :
Me représente un atome de titane, de zirconium ou d'hafnium tétravalent ;
$R^1$ représente un groupe alkyle, alcényle ou alcynyle linéaire ou ramifié, un groupe cycloalkyle ou cycloalcényle éventuellement substitué par un ou des groupes alkyle en $C_1$-$C_4$, ou représente un groupe aryle, alkylaryle, aralkyle, alkylaralkyle, aralcényle, alkylaralcényle, aralcynyle ou alkylaralcynyle, tous groupes qui sont éventuellement substitués une ou plusieurs fois par des groupes : $(C_6H_5)_2P$-, $(R^5O)_2P(O)$-, $R^5_3$ Si-, $R^5_3$ SiO-, $R^5SO_2$-, -S-(alkylène en $C_2$-$C_4$-S)-, -O-(alkylène en $C_2$-$C_4$)-O-, où $R^5$ représente un groupe phényle, benzyle ou alkyle en $C_1$-$C_8$, ou par un groupe cyano, F, nitro, alkylthio en $C_1$-$C_{12}$, alcoxy en $C_1$-$C_{12}$, amino secondaire ou -$COR^4$, où $R^4$ représente le reste d'un alcool

monovalent ; ou représente un reste d'un énol, d'une énamine ou d'une énhydrazine ;
$R^2$ représente un groupe cyclopentadiényle, alcoxy, cycloalcoxy, cycloalkylalcoxy, aryloxy, aralkyloxy, alkylthio, arylthio ou aralkylthio éventuellement substitué par un ou des groupes alkyle, alcényle, alcoxy, cycloalkyle, aryle, aralkyle, trialcoxysilyle, trialkylsilyle ou des atomes d'halogènes ; ou représente un atome d'halogène, un pseudohalogène, un groupe acyloxy, acylamino ou trialkylsilyloxy,
$R^3$ représente le reste, diminué d'un atome d'hydrogène, d'un groupe hydroxyle, thiol ou amino, d'un sucre, thiosucre ou amino sucre optiquement actif, protégé, et présentant une fonctionnalité monohydroxy, monothiol ou monoamino, ou bien un tel reste de leurs dérivés du groupe des alcools de sucres ; ou un reste d'ester d'un acide saccharique, aldosaccharique, ou cétosaccharique ; un reste d'un aminosucre, d'un sucre-mercaptal ou d'un désoxysucre,
x vaut 0, 1 ou 2 et y vaut 0, 1, 2 ou 3, et
$M^{\oplus}$ représente $Li^{\oplus}$, $Na^{\oplus}$, $K^{\oplus}$, $MgY^{\oplus}$, $ZnY^{\oplus}$, $CdY^{\oplus}$, $HgY^{\oplus}$, $CuY^{\oplus}$ ou représente un ammonium quaternaire, Y représentant un atome d'halogène, caractérisé en ce que l'on fait réagir un composé de formule II :

$$R^2_xR^3_{3-x}MeX \qquad (II)$$

ou un composé de formule IIa :

$$R^2_yR^3_{4-y}Me \qquad (IIa)$$

en présence d'un solvant inerte et d'un gaz protecteur inerte, avec un composé de formule III :

$R^{1\ominus}M^{\oplus}$ (III) ,

Me, $R^1$, $R^2$, $R^3$, $M^{\oplus}$, x et y étant définis comme ci-dessus, et X représentant un anion.

**2.** Procédé selon la revendication 1, dans lequel $R^1$ représente un groupe alkylaralcynyle en $C_9$-$C_{16}$, aralcynyle en $C_8$-$C_{16}$, alkylaralcényle en $C_9$-$C_{16}$, aralcényle en $C_8$-$C_{16}$, alkylaralkyle en $C_8$-$C_{16}$, alkylaryle ou aralkyle en $C_7$-$C_{16}$, aryle en $C_6$-$C_{12}$, cycloalcényle avec de 3 à 8 atomes de carbone dans le cycle, cycloalkyle avec de 3 à 8 atomes de carbone dans le cycle, alcynyle en $C_2$-$C_{12}$, alcényle en $C_2$-$C_{12}$ ou alkyle en $C_1$-$C_{18}$, linéaire ou ramifié, éventuellement substitué une ou plusieurs fois par des groupes amino secondaire, cyano, nitro, alkylthio en $C_1$-$C_6$, alcoxy en $C_1$-$C_6$ ou $-COR^4$, où $R^4$ représente un groupe alcoxy en $C_1$-$C_{12}$ ; ou représente un reste d'un énol, d'une énamine ou d'une énhydrazine fixé par l'atome d'oxygène de l'énol ou l'atome d'azote de l'énamine.

**3.** Procédé selon la revendication 2, dans lequel $R^1$ représente un groupe (alkyle en $C_1$-$C_7$)-phénylpropargyle, ou (alkyle en $C_1$-$C_8$)phényléthynyle, (alkyle en $C_1$-$C_8$)phénylvinyle, phénylvinyle, phényléthynyle, ou phénylpropargyle, (alkyle en $C_1$-$C_8$)phényl(alkyle en $C_1$-$C_2$), phényl(alkyle en $C_1$-$C_2$), (alkyle en $C_1$-$C_{10}$)phényle, phényle, cycloalcényle avec de 3 à 6 atomes de carbone dans le cycle, cycloalkyle avec de 3 à 6 atomes de carbone dans le cycle, alcynyle en $C_2$-$C_6$, alcényle en $C_2$-$C_6$, alkyle en $C_1$-$C_6$, linéaire ou ramifié, éventuellement substitué une ou plusieurs fois par des groupes amino secondaire, cyano, nitro, alcoxy en $C_1$-$C_6$, alkylthio en $C_1$-$C_6$, ou $-COR^4$, où $R^4$ représente un groupe alcoxy en $C_1$-$C_{12}$ ; ou représente un reste d'un énol, d'une énamine ou d'une énhydrazine comportant jusqu'à 20 atomes de carbone, fixé par l'atome d'oxygène de l'énol ou par l'atome d'azote de l'énamine.

**4.** Procédé selon la revendication 2, dans lequel $R^1$ représente un groupe diméthylphényléthynyle ou -propargyle, méthylphényléthynyle, phénylpropargyle, phényléthynyle, méthylphénylvinyle, phénylvinyle, méthylbenzyle, 1-phényléthyl-2, benzyle, méthylphényle, phényle, cyclopentényle et cyclohexényle, cyclohexyle, cyclopentyle, propargyle, éthynyle, allyle, vinyle, alkyle en $C_1$-$C_4$, linéaire ou ramifié, éventuellement substitué une ou plusieurs fois par des groupes amino secondaire, cyano, nitro, alcoxy en $C_1$-$C_6$, alkylthio en $C_1$-$C_6$ ou $-COR^4$, où $R^4$ représente un groupe alcoxy en $C_1$-$C_{12}$ ; ou représente

un reste d'un énol, d'une énamine ou d'une énhydrazine comportant de 2 à 16 atomes de carbone, fixé par l'atome d'oxygène de l'énol ou par l'atome d'azote de l'énamine.

**5.** Procédé selon la revendication 1, dans lequel $R^2$ représente un groupe aralkylthio en $C_7$-$C_{16}$, arylthio en $C_6$-$C_{12}$, alkylthio en $C_1$-$C_{18}$, aralkyloxy en $C_7$-$C_{16}$, aryloxy en $C_6$-$C_{12}$, alcoxy en $C_1$-$C_{18}$, ou cyclopentadiényle, éventuellement substitué par des groupes alkyle en $C_1$-$C_6$,alcényle en $C_2$-$C_6$, alcoxy en $C_1$-$C_6$, cycloalkyle comportant 5 ou 6 atomes de carbone dans le cycle, phényle, benzyle, trialcoxysilyle comportant de 1 à 6 atomes de carbone dans les groupes alcoxy, trialkylsilyle comportant de 1 à 6 atomes de carbone dans les groupes alkyle, ou par des atomes de F, Cl ou Br ; ou représente un atome d'halogène, ou un groupe pseudo-halogène, acyloxy en $C_1$-$C_{18}$, acylamino en $C_1$-$C_{18}$ ou trialkylsilyloxy comportant de 1 à 6 atomes de carbone dans les groupes alkyle.

**6.** Procédé selon la revendication 5, dans lequel $R^2$ représente un groupe benzylthio, phénylthio, alkylthio en $C_1$-$C_6$, benzyloxy, phénoxy, alcoxy en $C_1$-$C_6$ ou cyclopentadiényle, éventuellement substitué comme indiqué dans la revendication 5 ; ou représente un atome de Cl, Br, I, ou un groupe CN, CNS, CNO, acyloxy en $C_1$-$C_{12}$,acylamino en $C_1$-$C_{12}$ ou trialkylsilyloxy comportant de 1 à 4 atomes de carbone dans les groupes alkyle.

**7.** Procédé selon la revendication 1, dans lequel $R^3$ représente le reste d'un monosaccharide en $C_3$-$C_7$ présentant une fonction monohydroxy, monothiol ou monoamino, protégé, ou bien un tel reste de di- ou trisaccharides correspondants, ou de leurs dérivés du groupe des alcools de sucres ; ou d'un ester d'un acide saccharique, d'un acide aldosaccharique, ou d'un acide cétosaccharique ; ou d'un aminosucre, d'un désoxysucre, ou d'un sucre-mercaptal.

**8.** Procédé selon la revendication 7, dans lequel $R^3$ représente le reste d'un monosaccharide en $C_5$ ou $C_6$, ou d'un de ses dérivés.

**9.** Procédé selon la revendication 7, dans lequel $R^3$ représente le reste d'un pyranose ou furanose protégé.

**10.** Procédé selon la revendication 1, dans lequel les groupes hydroxy des sucres et de leurs dérivés sont protégés par des groupes acyle en $C_1$-$C_8$, alkyle en $C_1$-$C_8$, benzyle, diphénylméthyle, trityle, alkylidène en $C_1$-$C_8$,triphénylsilyle ou trialkylsilyle comportant de 1 à 8 atomes de carbone dans les groupes alkyle, ou $(C_6H_5)_2Si=$, (alkyle en $C_1$-$C_8$)$_2Si=$, $(C_6H_5)_2Sn=$ ou (alkyle en $C_1$-$C_8$)$_2Sn=$.

**11.** Procédé selon la revendication 1, dans lequel $R^3$ représente le reste, diminué de l'atome d'hydrogène du groupe hydroxyle, du 1,2:5,6-di-O-isopropylidène-α-glucofuranose.

**12.** Procédé selon la revendication 1, dans lequel x vaut 1 et y vaut 1.

**13.** Procédé selon la revendication 1, dans lequel Y représente un atome de Cl, Br ou I.

**14.** Procédé selon la revendication 1, dans lequel $M^{\oplus}$ représente un ion $Li^{\oplus}$, $MgCl^{\oplus}$, $MgBr^{\oplus}$, $ZnCl^{\oplus}$, $ZnBr^{\oplus}$,$CdCl^{\oplus}$, $CdBr^{\oplus}$ ou tétraalkylammonium comportant de 1 à 6 atomes de carbone dans les groupes alkyle.

**15.** Procédé selon la revendication 1, dans lequel Me représente un atome de Ti, $R^1$ représente un groupe allyle ou un groupe 1-(t-butyloxy)-vinyl-1-oxyle, $R^2$ représente un groupe cyclopentadiényle, et $R^3$ représente le reste, diminué de l'atome d'hydrogène du groupe hydroxyle, du 1,2:5,6-di-O-isopropylidène-α-glucofuranose.

**16.** Procédé pour la préparation des composés de formule II :

$$R^2_xR^3_{3-x}MeX \qquad (II)$$

dans laquelle Me, $R^2$, $R^3$ et x sont tels que définis dans la revendication 1, caractérisé en ce que l'on

fait réagir 1 mole d'un sel de formule :

$R^2_x\ MeX_{4-y}$

avec 1 à 3 moles d'un sucre, thiosucre ou aminosucre optiquement actif, présentant une fonctionnalité monohydroxy, monothiol ou monoamino, protégé, ou de leurs dérivés appartenant au groupe des alcools de sucres ; d' un ester d'un acides saccharique, aldosaccharique ou cétosaccharique ; d'un aminosucre, d'un sucre-mercaptal ou d'un désoxysucre de formule $R^3H$.

**17.** Utilisation des composés de formules I ou Ia selon la revendication 1 comme réactifs énantiosélectifs pour des composés comportant des groupes carbonyle et/ou imine N-substitués.

**18.** Procédé de préparation d'alcools secondaires et tertiaires et d'amines secondaires, caractérisé en ce que l'on fait réagir des aldéhydes, cétones, aldimines ou cétimines N-substituées avec 1 mole d'un composé de formule I ou Ia selon la revendication 1, par mole de groupe aldéhyde, céto ou imine.

**19.** Procédé selon la revendication 18, caractérisé en ce qu'il est mis en oeuvre en présence d'un solvant inerte et de gaz protecteurs inertes.

**20.** Procédé selon la revendication 19, caractérisé en ce qu'il est mis en oeuvre à des températures de -80 à 30 °C.